# EUROPÄISCHE PATENTANMELDUNG

(11) **EP 2 103 692 A1**
(43) Veröffentlichungstag der Anmeldung: **23.09.2009**
(21) Anmeldenummer: 08153118.8
(22) Anmeldetag: 20.03.2008
(51) Int. Cl.: C12Q 1/68

(54) **Genveränderungen im humanen PLK1-Gen**

(71) Anmelder: Universität Duisburg-Essen, 45141 Essen (DE)
(72) Erfinder: Riemann, Kathrin, 45145, Essen (DE); Siffert, Winfried, 45884, Gelsenkirchen (DE)
(74) Vertreter: Helbing, Jörg

(57) **Zusammenfassung**

Die Erfindung betrifft ein *in-vitro* Verfahren zur Vorhersage des Verlaufs von Krebserkrankungen, des Ansprechens auf pharmakologische oder nichtpharmakologische Therapiemaßnahmen zur Behandlung dieser Erkrankungen und von unerwünschter Arzneimittelwirkungen umfassend die Detektion von Genveränderungen im humanen Gen *PLK1*, das für das Protein Polo-like Kinase 1 kodiert. Im Einzelnen wird eine Genveränderung in der 3'-untranslatierten Genregion von *PLK1,* nämlich ein Polymorphismus an Position 11.285, bei dem ein Adenin durch ein Guanin substituiert ist, beschrieben, der sowohl für die obigen Vorhersagen als auch zum Auffinden und Validieren weiterer für die oben genannten Zwecke verwendbare Genveränderungen geeignet ist. Weiterhin werden Kits für diese Verfahren zur Verfügung gestellt.

SEQUENCE LISTING

<110>  Universitaet Duiburg-Essen

<120>  PLK1 Polymorphismus

<130>  080015ep

<160>  9

<170>  PatentIn version 3.3

<210>  1
<211>  12600
<212>  DNA
<213>  Homo sapiens

<400>  1
gagcggtgcg gaggctctgc tcggatcgag gtctgcagcg cagcttcggg agcatgagtg      60

ctgcagtgac tgcagggaag ctggcacggg caccggccga ccctgggaaa gccggggtcc     120

ccggagttgc agctcccgga gctccggcgg cggctccacc ggcgaaagag atcccggagg     180

tcctagtgga cccacgcagc cggcggcgct atgtgcgggg ccgctttttg ggcaagggcg     240

gctttgccaa gtgcttcgag atctcggacg cggacaccaa ggaggtgttc gcgggcaaga     300

ttgtgcctaa gtctctgctg ctcaagccgc accagaggga gaagatgtcc atggaaatat     360

ccattcaccg cagcctcgcc caccagcacg tcgtaggatt ccacggcttt ttcgaggaca     420

acgacttcgt gttcgtggtg ttggagctct gccgccggag ggtgagtgtc gctgctgggg     480

aactggaact gcctgcgggg cagttggagc gcccagacct ggagctgctg gaaagagtac     540

ccagcaaggg agagcctggg acttggagct gctagagaag ggtgctggga gcctcccgct     600

tacgtatgga aagtgtcttt ggtaagggct tcttggctct gggagctgct agaggagggg     660

gttccagtga agtggagtct gagtcatgtt gctgataggg aagtcagctt ctggacgcga     720

ggtgctgggg ggaggggtgc tggtaatgga cgctagggcc ctggagtacc cagggaggag     780

tcccaggtta gtgatgccgc gccctgggag cttctggggt ggagctgggt cagtgggggg     840

gtagttggag ccagacttcg ggccttccgg gggagatacg agtcaggaaa gggatctggt     900

gctggggact cggagcttcc ggagtggggc tgagagagga cccccaggta aggggggaag     960

ctagtaggag aaggggtgct gcgaatggtt gtggacagtg ttaaggcagg gtccagatgc    1020

cgctgtgctg gagaaggaat ggggtggggg catagggaag gagagaaacc caccaagacc    1080

cctctttcat cccttgggag tccagagtcc agtcctgtgc ttcctttgcc tggtaaccct    1140

ctcccttccc caccggcctc aatccacctc cccatccctc ctgccctctc cttcccaccc    1200

acagtctctc ctggagctgc acaagaggag gaaagccctg actgagcctg aggcccgata    1260

ctacctacgg caaattgtgc ttggctgcca gtacctgcac cgaaaccgag ttattcatcg    1320

agacctcaag ctgggcaacc ttttcctgaa tgaagatctg gaggtgaaaa taggtgagtt    1380

gctgagcctg caggggtgct tgacatcact acaagaggct ggaatttgga ggaggctggg 1440

agaaaggaag gagacaaccc acaagtcagt atcttgccta cagatgcatt atttttttgt 1500

gccccaatgc aatatttttt ttttacaaag aatttgaatt tgtttttatt ttttaaattg 1560

gaaggtttga ttgaacaaaa agtttgattc agaactcctt ttaaacaggt gaaagcttca 1620

gtaatacgag gacagcattt tcttagggca gccctttgct gggctggaaa acagctgtcc 1680

ccttcagact tctttgccac agtctctact cctccctatt cttcacagac ttgccaagtc 1740

ttggaggtta acttgcttga tccttgaagg cattggagtt ttactatccc tgtcagttcc 1800

tgtttcaaag catagcccct ttctaaaagg aggggtgaga agtgtcactg gaggcacctc 1860

cctggtgtca gcatgggagg aagattcctg ggcaagcctt gctacataca aggagcagag 1920

gcttgtggga tcagatggcc ctggttctgg atggtcaaac cttaactagc cttctgcatt 1980

gacagatgtc agaggctggc atctaagtac caactcttcc tccctctgtc ccaggggatt 2040

ttggactggc aaccaaagtc gaatatgacg gggagaggaa gaagaccctg tgtgggactc 2100

ctaattacat agctcccgag gtgctgagca agaaagggca cagtttcgag gtggatgtgt 2160

ggtccattgg gtgtatcatg taagttggga gttgtctctg gaccaacctg gtctcagcag 2220

gggcaacttt gggacatcct acctggctga ccttttctgt ggacctttcg gcctgcttta 2280

cagaaagcct actccaaggg acaagtcatc cccaaacaaa gcctaattgt catatctttc 2340

cctgtcactg gtgtatcctg tcggatttct gcagcctagg tcatgggtac agcggaggag 2400

gaggagggag agtctccaca accttactgt agtcctttgc tagagttgtc cacagggcag 2460

catcaccatc atctgggaac ttgtatacac aatctcagga cccagactac ttactggatc 2520

agaatctgga tttttaacaa ggtcccttgg tgattccctt gcacaataag gtcttaaaca 2580

ccgatgagca ttagcatggt gggggaaatt aggccaggag ttacagatgg aaagatgatg 2640

tgccagtggt ctgtctggat taagaccatc agcagcggtt ggtggcaggc agaggcttgg 2700

gccttttcag gtgttatctt tcttgttttg ggcctaggta ctggagagtt agaaatgagt 2760

tgtagcaatg gctttcatac gtctttgcag tggggttttt tcaaatgaaa acttgaagac 2820

ttaagtacag aactgctaaa ggtggagctg cttctgtaag cgcaggtagc cctttgacct 2880

gttttgcatg acaggcagca tctgaggccc ttgcacagag tcctagatga ccacaggcta 2940

tagctaaaaa atcactggcc cttaagaatc ctggtctcac catgtgtatt ctgctgaggg 3000

cttattctag ccaatgtcat ggcttctggg gctgctcagt ggacttaggg attgtcttca 3060

ggggccccag agttagagtg agtgtccaga ggatgcctga cctttgttct gaccctgaga 3120

tgatttctct catgtctggg ttgtggctgg gagactggtg ccaaatccta ccttgtgctt 3180

acaggtatac cttgttagtg ggcaaaccac cttttgagac ttcttgccta aaagagacct 3240

acctccggat caagaagaat gaatacagta ttcccaaggt gactaatgat gctttaagtt 3300

tacatttatt ttgttttccc cagaagctgt ttatggagcc cagcaatatc ctaggaccag 3360

aaataggtct atcccacctc taaggtagcc acagagcttg agagggcctg tgtctgaaat 3420

tgcatacaga acttgacatg tatgtgcaca taagcatttt ttctaggggg agagagttgg 3480

cagattctca agagattggg taacccaaaa gttgtgtaaa aagcaaaaaa caaaggaggt 3540

aggaaggagt aggaggcaaa agtgcttaat ctttagaaat ggtttatctt aattcaaatg 3600

gattctgcca cttagcgaga tcacattggg caaatggcct gcatcttttt tttttttttt 3660

tttatttgag gtggagtttt gctcttgttg cccagtgcag tggcatgatc ttgactcact 3720

gcaacctcca cctcccaggt tcaagcgatt ctcctgcctc agcctcccaa gtagctggga 3780

ttacaggcac ccaccacctc gcctggcttt tttttttttt ttttgtattt ttagtagaga 3840

cggggtttca ccatgttggc caggctggtc tcgaactcct gacctcaggt gatccacccg 3900

ccttggcctc ccaaagtgct gggattacag gcgtgagcca ccatacccag cctggcctgc 3960

atcttctgaa ccatagtttc catatataaa gtggagttcc tgccttggag cacctcacag 4020

agtgccttgc atgtggtgag tgctctcaga attatgaatc actgtgaatc ccagggcttc 4080

ctaatggcat agttgtgtgc attttctttt tttttttttt tttttttttg agatggagtc 4140

tcgctctgtc gcccaggctg gaatgcagtg gtacaatctc ggctcactgc aagctctgcc 4200

tcccaggttc atggcattct cccgcctcag cctcctgagt agctggaact acaggcacca 4260

gccaccatgc ccggcttctt ttttctgtat ttgtggtaga gacagggttt gaccgtgtta 4320

gccaggatgg tctcgatctc ctgaccttgt gatccacctg cttcggactc cctaagtgct 4380

gagattacag gcgtgagcca ccgcgcccgg ccatggtgtg cattttcaca accaccaaca 4440

gaaatacatt taaaggaata gctggttcct gcagaagcct agcacacagc cagtttgaag 4500

tgtagtgtcc ttgtgtgaag ccaggaggcc tgtcaccata aggacagaca tagtaaatcc 4560

atcacaaagt gtcagttcac tgtcagagac agcatggaat caagccagct agacaaggcc 4620

ttcaccctct ctgtgcctca ctttcctcct ctgtgaaaaa gggatgataa tagatcccca 4680

ccccttagat gagttggtga gggctaagtg agctaaacac aggagtgctt agaatgcttt 4740

cctggcacat agtgagtgct aggaaagtga gttggtgttc ggccacagtc cattgaccct 4800

tctgtcaatg gtgctcagta aagctgacag tggagaactt ggcattgaac caagttgtga 4860

accactgacc tgtggtgtat ttgagactgg gggctgcatg tgggctgggg acctgcagct 4920

cctttgaggc cgtactgtac tccaggtccc cttcacattc tgcttatggc tgtccctctc 4980

tctgccccag cacatcaacc ccgtggccgc ctccctcatc cagaagatgc ttcagacaga 5040

tcccactgcc cgcccaacca ttaacgagct gcttaatgac gagttcttta cttctggcta 5100

tatccctgcc cgtctcccca tcacctgcct gaccattcca ccaaggtttt cgattgctcc 5160

cagcagcctg gaccccagca accggaagcc cctcacagtc ctcaataaag gtacaacaag 5220

ggtctgggta agagagcaga ccccccagag aaagcccagg ttgtaggggt gtgtgcagct 5280

tagtccctgg ccctgagagc tcaggtgtgg agtaggacag gcctctgtcc ttcaatccgt 5340

ggttccaatg cccatctgct tctcggccct gccaggaaag actgacctta ccatggctgg 5400

ataaacgtac catgcccaag aagaagaaaa tggattgaat gccaagcctg aaaaattctt 5460

tcaaatcact ttaattaatt taattttttg gagacagggt ctctcctcta ttttccaggt 5520

tggagtgcag aggcacaatc acagctcact gcagctttca actctagggc tcaattgatc 5580

ctcccatctc agcctcccaa gtagctggga ctgtcaggca tgcaccacca tgcctggctg 5640

acttttctaa tttttgtaga gacagtgttt tgccatgttg cccaggctgg tcttggaact 5700

cctgggctca agcgatcctt tcacctccac caaagtgttg ggatgacagg catgagctgc 5760

tacatctggc ctttaaaaaa atttctaaca ctttttcatg attcattgaa aaacataaaa 5820

atttcctttt ttttgtggcg tgatcccggc tcactgcaag ctctgcctcc cgggttcacg 5880

ccgttctcct gcctcagcct cctgagtagc tgggactaca ggcgcccacc accatgcccg 5940

gctgtttttt tgtatctttt agtagagacg gggtttcact gtgttagcca ggatggtctc 6000

aatctcctga cctcgtgatc cgcctgcctc ggcctcccaa agggctgaga ttacaggcgt 6060

gaaccaccag gcccggcctt tttttttttt tttttttttt tttttttttt tttttttttt 6120

gagacagggt ctggctctgt ctcctaggct ggaggggagt ggtatgatct cgatttcact 6180

aaggatacat agttttaaaa agtcacttga aatacttttt ttctctgtgg atatatcacc 6240

aatttgatgt acaacttcag ttcatttttc agttttcaat ttttaggtat tggtttttct 6300

taaacttcat tttggaataa ttttaggctt atacagaaaa gttgcaacaa caacaaaaaa 6360

ctccccagag ttcctacata ccttgcagca attattacag tggaattttt tttgagacag 6420

gtctcaggac ttgctgtcac ccaggctgga atgcagtggc tcactcatag cttactgcag 6480

ccccaacatc ctgggctcaa acagtccccc tgccttaccc tccagagtag ctaggactac 6540

aggcatgtgc caccataccc agctaattaa aaaatttttt tctttgggag gccgaggtgg 6600

gtggattgcc cgagctcagg agttcgagat cagcctgggc aacatggcga aacgctgtct 6660

ctactaaaaa tacaaaagac tagccgggcg tggtggtgtg cacctgtaat ctcagctact 6720

cgggacactg aggcacaaga atcacttgaa cctggaaggc ggaggttgca gtgagccgag 6780

gtctcaccac tgcactccag cctgggcaac agagcgagac tctgtctcaa aaaaaaaaaa 6840

aaattttttt tttgtaaaga caagatctca ctatgtcgac caggcttatc tcaaactcct 6900

gccctcaagt gatcctcctc ggcctcccaa agtgttagga tttacaggtg tgagccactg 6960

tgccctgcct agaatgatgt tctaatggta atttttcttt tcctcacttt ttttgcattt 7020

aataattgga attcttctgt aaggaagagc tgttccttct ctccgattga tttatttatt 7080

tggttatgtc tgaaatagac tcatggatat ttttattctt tgggctatat tttaaaggat 7140

tacttatttc tctgctttta tttatttttt taaatttgag acagggtctt gctctgtctc 7200

ccgggctgga gtgcagtggc acagtcgtac ctcacagtag ctttgatctc ctgggctcaa 7260

gtgatcctcc agcctcagcc tcccaagtag ttgagacttt aggcatgtgc caccgcgtct 7320

gaccaatttt taaatttttg gtagagatga ggtcttgccc acactggtct caaactcctg 7380

agctcaagtg atcctcctgc ctcagactcc tacttttttt ctttcagttt taatttttta 7440

tatagcattt taaaaactta aggaaatttt cagttacccg aaggtggaga gaactgtatg 7500

atgagtccca tgtttcatct atttttacta gttatcaaca tttggacaga ctgttttatt 7560

tattccagcc tcctgccttt tttgttaccg gtgttttaaa aaattattat ttactattta 7620

ttattattta tttgagacat tcttgctttg ttgcccaggc tggagtgctg tggcaagatc 7680

atggctcact gcagcctcca cctcctgcgt tcaagtgact ctcccacctc agcctcccaa 7740

gtagctagga ctacaggtgt gccaccatgt ccagctagtt tctgtatttt tagtaaagac 7800

agggttttgc catgttgctc aggctggtct caaactcctg agctcaaaca atctacctgc 7860

ctcggtctct catagtgttg ggattacaag tgtgagccac tgggcctggc cttgctggag 7920

tgtttaaagc aaatccctaa tatcttatta tttcataccc aagtccctat catgacatcg 7980

ttttttaatt gaaaacttaa acattttata ttatacaagg agaattagca catggtggtg 8040

ggggaataaa taaataagga aaaagacaat aagaataatc tgtgtttcta ctgcccagat 8100

ataatcagcg ttaaaatttc atgttatctt ttcaagcttt tttggttata aataagcata 8160

taaagcaaat tttggggtgg aaaaaaattt cttgtgtttt tatttaaatg cctactgaat 8220

ccattctgtg gatgaactga actactcagc ccttccatct gcctggatgc tgaagatgct 8280

ttccattctt tgctcaaagc tgggcagctc tgagctgctg gtgccttcaa gctgtttgcc 8340

tgagtggcag gtcatcctgc agggccacac cgttgggtca gaggccacgg cttctgaaga 8400

tctgttgcct cacagtgagc actatgtgcc tgtcacctga gcatggcagc tgctgctcta 8460

gtaaccaggc actgattcag tttccccaaa gcagtggtag ctaagagaat ttttctttcg 8520

gaagagtttc agctgtggca ggggagtccc gtgcccttcc caacgcccct gtttttgtca 8580

ccttcctagg cttggagaac cccctgcctg agcgtccccg ggaaaaagaa gaaccagtgg 8640

ttcgagagac aggtgaggtg gtcgactgcc acctcagtga catgctgcag cagctgcaca 8700

gtgtcaatgc ctccaagccc tcggagcgtg ggctggtcag gcaaggtggg tactgcgggg 8760

ccctgggcgg ggcaggattg cttggggcat ctggaaaagg caggaggagg cttggccaac 8820

aaagcactgg tgacttgttc aggccctgtc tgcataggac cattggtttc cccatttcct 8880

gatatccaag gccctgcttc cgtggctcct gccgcctttt ctgatccgtc ttttttactc 8940

tagcacctcg atgtgccacc ttcatacgct gtttcttttg ctcagtaagt tcctattgta 9000

tcttcaagac ccttgaaata acagacccag atgtactgca gcctcagggc tttctccctg 9060

gtcctggcac gaaagcactt cttccagaca ccacatggct cacactgtgg cctccttcaa 9120

gtgtctgctc cagtgtcagc tctgtgagca tctccctggc ccctctgctg gtgcttctca 9180

tcctcttgcc cactgcatag ttctccataa cacttctcac ccaacatagt ccaacatgtc 9240

cttctttgtt tattggattt aaattaaggg gtttgttgca tgcactgaac gtaacatttt 9300

gatgtgtttg caactggctt ctgtcttcac agtttcttaa ttactccaaa ctggaaaact 9360

agatgcctct gactcttacc atgatagaag tcactgatat tttgcccata cttaaattga 9420

ttagtctttg cccctacaac cacagtgtaa gctccgtgga ggctggggtt ttgtctgttg 9480

ctcactgaac ctgcaacagt gcctggcacg gtaggccttc agtaaatggg tcagtggaat 9540

gaaggagtga gtggaacttg gtgcgctgtg ctgagacttt ctctgcagag atatctgtgc 9600

tgctgcttgt ctgagcctgc tggcagcaga ggccctgctt tgctcttctc tggggcccta 9660

ggcctctcaa ctgagcccag gtggggtgcc cagcaggctt ccctgttccc tggtgtgggc 9720

cacatgtgtg gagcagaggg gaagaggctg gtcctgacca actaactgtc tgtctgtttc 9780

tgtctcagag gaggctgagg atcctgcctg catccccatc ttctgggtca gcaagtgggt 9840

ggactattcg gacaagtacg gccttggtag gtttcttcca gaacaggtgg gtgactcagg 9900

cacagccagg tgaccttttc agttgttaca gactctggcc tttttgagct cccaggtact 9960

gttctcagtg ccctcctctc tccatcccag gcctcccagt tccagctccc agtgctccct 10020

gactccccag cttttttttt ttccagagac agggtcctgc tctgtagccc aggctggagt 10080

gcagtggcac aatcatggct cactgcagcc tcaaactccc aggcccctaa gtagctgtga 10140

ctacaggcgt gcaccattat gctcagctaa tttttaaaat attttgtaga gatggggtct 10200

cattacgttg cccaggctgg tctcaaactc ctgggctcaa acaatcctcc tccctcagcc 10260

tcccaaagtg ctggaatcac aggcatgtgc caccacgccc ggtcccactc cccactttct 10320

attccccctt tctgagacct ctctccaccg atccctaggg tatcagctct gtgataacag 10380

cgtgggggtg ctcttcaatg actcaacacg cctcatcctc tacaatgatg gtgacagcct 10440

gcagtacata gagcgtgacg gcactgagtc ctacctcacc gtgagttccc atcccaactc 10500

cttgatgaag aaggtgagtg ccgtccggcc catggggggt ggtgttgcag aagtgggacc 10560

tgtgctggag gatcagactc taattctgga accccttacc tacttttcat ccagatcacc 10620

ctccttaaat atttccgcaa ttacatgagc gagcacttgc tgaaggcagg tgccaacatc 10680

acgccgcgcg aaggtgatga gctcgcccgg ctgccctacc tacggacctg gttccgcacc 10740

cgcagcgcca tcatcctgca cctcagcaac ggcagcgtgc agatcaactt cttccaggtg 10800

agctggaggt caccaggcgc aggagagagc tggggtaggc tccgcatgcc tggcagtggc 10860

ccatgtgggt tgaatgtgga gtgagcggct caggtaccta taacctgttg tgtcttccct 10920

ctactcccta acatacactg gcctctggga tcgccaaccc ctgctgctct tctcttgcag 10980

gatcacacca agctcatctt gtgcccactg atggcagccg tgacctacat cgacgagaag 11040

cgggacttcc gcacataccg cctgagtctc ctggaggagt acggctgctg caaggagctg 11100

gccagccggc tccgctacgc ccgcactatg gtggacaagc tgctgagctc acgctcggcc 11160

agcaaccgtc tcaaggcctc ctaatagctg ccctcccctc cggactggtg ccctcctcac 11220

tcccacctgc atctggggcc catactggtt ggctcccgcg gtgccatgtc tgcagtgtgc 11280

cccccagccc cggtggctgg gcagagctgc atcatccttg caggtggggg ttgctgtata 11340

agttattttt gtacatgttc gggtgtgggt tctacagcct tgtccccctc cccctcaacc 11400

ccaccatatg aattgtacag aatatttcta ttgaattcgg aactgtcctt tccttggctt 11460

tatgcacatt aaacagatgt gaatattctt tttcttgtat ttcctgaggg gtgccagggc 11520

ctgggatcca gcgaacatct ctgcttcatc agccccaggc tgcccagcct ctgccagtct 11580

tgtgggggaa agggggtgac agtgtctctc tgtggaccag gctggagtgc agtggcatga 11640

tcctggctca ctgcagcctc gaactcctgg gctcaagtga ttctcccacc tcagcctccc 11700

aagcagctgg gactacaggc gtgcgccacc atgcctggct aattttacaa attttttgta 11760

gaaatggggt gttgccatgt tggccaggct ggtctcgaac tcctgagctc aagtgatcct 11820

ctcactcagc ctcccaaaat gctgggatta caggtgtgag ccactgcacc cagcctgatt 11880

ctgaggccag ccacaggctc agctcttcag tgagccagca cggagaccat ctgtgtggca 11940

tccagcccac ctcacctccc tgtggcccca gggcatggcc tcctggcctc tgagtctggc 12000

gggtagtagg gcaggaagag gctctcagag gcgcagctcc tcatggctcg gtgcgtgtgg 12060

aggagcagcc gtgggaagcg gtttgtgaag tactggacga agccatcagg gacttggccg 12120

agtgcctgtc gcacctcaac tgggagctcc ctgtagtggt gcttctgtgg gtaggtagag 12180

cagagaaccc tggctcagct gcggccaggc cttcccaaga cccaggccca cccaggcccc 12240

aacacatacc ttgttcctca cagcacggag caggtctcgc actgatgtcc ccttatagga 12300

ccggaacttt ctcagatctg tggacaggga attcagtggc aaaaccgtcc ctgctagact 12360

cccctccacc gcccaggccc actcccctcc accgcccagg cccacctgag tatggcctct 12420

acctgtctgc agcggcatgg agatgtgctc gtgccagttg tcccggacca ctgcgcagcc 12480

tcccgcctcc agtgccctca ccaggggctc ctgctcggac tccttctcca gccagtcact 12540

gacgtcctgg ggcccagaga gctcctgagc cttgtgaccg gggccagagg ccacatagcc 12600

<210> 2
<211> 2208

<212>  DNA
<213>  Homo sapiens

<400>  2
gagcggtgcg gaggctctgc tcggatcgag gtctgcagcg cagcttcggg agcatgagtg      60

ctgcagtgac tgcagggaag ctggcacggg caccggccga ccctgggaaa gccggggtcc     120

ccggagttgc agctcccgga gctccggcgg cggctccacc ggcgaaagag atcccggagg     180

tcctagtgga cccacgcagc cggcggcgct atgtgcgggg ccgctttttg ggcaagggcg     240

gctttgccaa gtgcttcgag atctcggacg cggacaccaa ggaggtgttc gcgggcaaga     300

ttgtgcctaa gtctctgctg ctcaagccgc accagaggga gaagatgtcc atggaaatat     360

ccattcaccg cagcctcgcc caccagcacg tcgtaggatt ccacggcttt ttcgaggaca     420

acgacttcgt gttcgtggtg ttggagctct gccgccggag gtctctcctg gagctgcaca     480

agaggaggaa agccctgact gagcctgagg cccgatacta cctacggcaa attgtgcttg     540

gctgccagta cctgcaccga aaccgagtta ttcatcgaga cctcaagctg ggcaaccttt     600

tcctgaatga agatctggag gtgaaaatag gggattttgg actggcaacc aaagtcgaat     660

atgacgggga gaggaagaag accctgtgtg ggactcctaa ttacatagct cccgaggtgc     720

tgagcaagaa agggcacagt ttcgaggtgg atgtgtggtc cattgggtgt atcatgtata     780

ccttgttagt gggcaaacca ccttttgaga cttcttgcct aaaagagacc tacctccgga     840

tcaagaagaa tgaatacagt attcccaagc acatcaaccc cgtggccgcc tccctcatcc     900

agaagatgct tcagacagat cccactgccc gcccaaccat taacgagctg cttaatgacg     960

agttctttac ttctggctat atccctgccc gtctccccat cacctgcctg accattccac    1020

caaggttttc gattgctccc agcagcctgg accccagcaa ccggaagccc ctcacagtcc    1080

tcaataaagg cttggagaac cccctgcctg agcgtccccg ggaaaaagaa gaaccagtgg    1140

ttcgagagac aggtgaggtg gtcgactgcc acctcagtga catgctgcag cagctgcaca    1200

gtgtcaatgc ctccaagccc tcggagcgtg ggctggtcag gcaagaggag gctgaggatc    1260

ctgcctgcat ccccatcttc tgggtcagca agtgggtgga ctattcggac aagtacggcc    1320

ttgggtatca gctctgtgat aacagcgtgg gggtgctctt caatgactca acacgcctca    1380

tcctctacaa tgatggtgac agcctgcagt acatagagcg tgacggcact gagtcctacc    1440

tcaccgtgag ttcccatccc aactccttga tgaagaagat caccctcctt aaatatttcc    1500

gcaattacat gagcgagcac ttgctgaagg caggtgccaa catcacgccg cgcgaaggtg    1560

atgagctcgc ccggctgccc tacctacgga cctggttccg cacccgcagc gccatcatcc    1620

tgcacctcag caacggcagc gtgcagatca acttcttcca ggatcacacc aagctcatct    1680

tgtgcccact gatggcagcc gtgacctaca tcgacgagaa gcgggacttc cgcacatacc    1740

gcctgagtct cctggaggag tacggctgct gcaaggagct ggccagccgg ctccgctacg    1800

cccgcactat ggtggacaag ctgctgagct cacgctcggc cagcaaccgt ctcaaggcct 1860

cctaatagct gccctcccct ccggactggt gccctcctca ctcccacctg catctggggc 1920

ccatactggt tggctcccgc ggtgccatgt ctgcagtgtg ccccccagcc ccggtggctg 1980

ggcagagctg catcatcctt gcaggtgggg gttgctgtat aagttatttt tgtacatgtt 2040

cgggtgtggg rsttctacag ccttgtcccc ctccccctca accccaccat atgaattgta 2100

cagaatattt ctattgaatt cggaactgtc ctttccttgg ctttatgcac attaaacaga 2160

tgtgaatatt cttaaaaaaa aaaaaaaaaa aaaaaaaaaa aaaaaaaa 2208

<210> 3
<211> 1800
<212> DNA
<213> Homo sapiens

<220>
<221> misc_feature
<222> (538)..(538)
<223> n is a or g

<400> 3
agctggaggt caccaggcgc aggagagagc tggggtaggc tccgcatgcc tggcagtggc 60

ccatgtgggt tgaatgtgga gtgagcggct caggtaccta taacctgttg tgtcttccct 120

ctactcccta acatacactg gcctctggga tcgccaaccc ctgctgctct tctcttgcag 180

gatcacacca agctcatctt gtgcccactg atggcagccg tgacctacat cgacgagaag 240

cgggacttcc gcacataccg cctgagtctc ctggaggagt acggctgctg caaggagctg 300

gccagccggc tccgctacgc ccgcactatg gtggacaagc tgctgagctc acgctcggcc 360

agcaaccgtc tcaaggcctc ctaatagctg ccctcccctc cggactggtg ccctcctcac 420

tcccacctgc atctggggcc catactggtt ggctcccgcg gtgccatgtc tgcagtgtgc 480

cccccagccc cggtggctgg gcagagctgc atcatccttg caggtggggg ttgctgtnta 540

agttattttt gtacatgttc gggtgtgggt tctacagcct tgtccccctc cccctcaacc 600

ccaccatatg aattgtacag aatatttcta ttgaattcgg aactgtcctt tccttggctt 660

tatgcacatt aaacagatgt gaatattctt tttcttgtat ttcctgaggg gtgccagggc 720

ctgggatcca gcgaacatct ctgcttcatc agccccaggc tgcccagcct ctgccagtct 780

tgtgggggaa agggggtgac agtgtctctc tgtggaccag gctggagtgc agtggcatga 840

tcctggctca ctgcagcctc gaactcctgg gctcaagtga ttctcccacc tcagcctccc 900

aagcagctgg gactacaggc gtgcgccacc atgcctggct aattttacaa attttttgta 960

gaaatggggt gttgccatgt tggccaggct ggtctcgaac tcctgagctc aagtgatcct 1020

ctcactcagc ctcccaaaat gctgggatta caggtgtgag ccactgcacc cagcctgatt 1080

ctgaggccag ccacaggctc agctcttcag tgagccagca cggagaccat ctgtgtggca 1140

tccagcccac ctcacctccc tgtggcccca gggcatggcc tcctggcctc tgagtctggc 1200

gggtagtagg gcaggaagag gctctcagag gcgcagctcc tcatggctcg gtgcgtgtgg 1260

aggagcagcc gtgggaagcg gtttgtgaag tactggacga agccatcagg gacttggccg 1320

agtgcctgtc gcacctcaac tgggagctcc ctgtagtggt gcttctgtgg gtaggtagag 1380

cagagaaccc tggctcagct gcggccaggc cttcccaaga cccaggccca cccaggcccc 1440

aacacatacc ttgttcctca cagcacggag caggtctcgc actgatgtcc ccttatagga 1500

ccggaacttt ctcagatctg tggacaggga attcagtggc aaaaccgtcc ctgctagact 1560

cccctccacc gcccaggccc actcccctcc accgcccagg cccacctgag tatggcctct 1620

acctgtctgc agcggcatgg agatgtgctc gtgccagttg tcccggacca ctgcgcagcc 1680

tcccgcctcc agtgccctca ccaggggctc ctgctcggac tccttctcca gccagtcact 1740

gacgtcctgg ggcccagaga gctcctgagc cttgtgaccg gggccagagg ccacatagcc 1800

<210> 4
<211> 60
<212> DNA
<213> Homo sapiens

<400> 4
gcagagctgc atcatccttg caggtggggg ttgctgtata agttattttt gtacatgttc 60

<210> 5
<211> 60
<212> DNA
<213> Homo sapiens

<400> 5
gcagagctgc atcatccttg caggtggggg ttgctgtgta agttattttt gtacatgttc 60

<210> 6
<211> 21
<212> DNA
<213> Artificial

<220>
<223> primer

<400> 6
tcttccctct actccctaac a 21

<210> 7
<211> 19
<212> DNA
<213> Artificial

<220>
<223> primer

<400>  7
ggacaaggct gtagaaccc                                                    19


<210>  8
<211>  19
<212>  DNA
<213>  Artificial

<220>
<223>  primer

<400>  8
ctcccgcggt gccatgtct                                                    19


<210>  9
<211>  24
<212>  DNA
<213>  Artificial

<220>
<223>  primer

<400>  9
ccgaacatgt acaaaaataa cgta                                              24

## Beschreibung

Die Erfindung betrifft ein *in-vitro* Verfahren zur Vorhersage des Verlaufs von Krebserkrankungen, des Ansprechens auf pharmakologische oder nichtpharmakologische Therapiemaßnahmen zur Behandlung dieser Erkrankungen und von unerwünschter Arzneimittelwirkungen umfassend die Detektion von Genveränderungen im humanen Gen *PLK1,* das für das Protein Polo-like Kinase 1 kodiert. Im Einzelnen wird eine Genveränderung in der 3'-untranslatierten Genregion von *PLK1,* nämlich ein Polymorphismus an Position 11.285, bei dem ein Adenin durch ein Guanin substituiert ist, beschrieben, der sowohl für die obigen Vorhersagen als auch zum Auffinden und Validieren weiterer für die oben genannten Zwecke verwendbare Genveränderungen geeignet ist. Weiterhin werden Kits für diese Verfahren zur Verfügung gestellt.

### Hintergrund der Erfindung

Ein wesentlicher Aspekt bei der Behandlung von Krebserkrankungen ist zunächst das Erstellen einer genauen Diagnose. Dies führt zu Kenntnissen darüber, in welchem Stadium sich die Krebserkrankung befindet, was auch über die nachfolgenden Therapiemodalitäten entscheidet, z.B. chirurgische Entfernung des Tumors mit ggf. Bestrahlung und pharmakologischer Therapie, eventuell auch unter Einsatz physikalischer Verfahren (z.B. Hyperthermie), Umstellung der Ernährung im Sinne einer spezifischen Ernährung für Krebspatienten. Das klassische Verfahren zur Diagnosestellung beinhaltet das sog. Staging und Grading. Das Staging beschreibt die Tumorgröße und die Invasivität und untersucht, ob Lymphknoten befallen sind und ob Fernmetastasen vorhanden sind (z.B. TMN-System). Beim Grading werden die Tumorzellen histologisch untersucht, wobei hoch differenzierten Tumorzellen ein niedrigeres Malignitätspotential zugeordnet wird als schwach oder entdifferenzierten Zellen. Obwohl dieses System sich in der Praxis bewährt hat, ist die individuelle Vorhersagekraft für den einzelnen Patienten limitiert, und es entspricht der klinischen Erfahrung, dass Patienten mit gleichem Tumorstadium deutlich unterschiedliche Krankheitsverläufe zeigen und unterschiedlich auf eine Therapie ansprechen, was sich letztendlich in drastisch unterschiedlichen Überlebenszeiten zeigt. Aus diesem Grunde ist es für die klinische Praxis erforderlich, zusätzliche und präzisere Marker bereitzustellen, die eine verbesserte Individualprognose einer Krebserkrankung erlauben. Dies kann geschehen über die Einbeziehung histochemischer Marker, wie dies beispielsweise beim Mammakarzinom im Rahmen der Untersuchung der Expression von Östrogenrezeptoren erfolgt. Ein alternativer Weg ist die Suche nach Gendefekten, z.B. somatischen Mutationen in Tumorsuppressorgenen. Neuere Ansätze versuchen den Krankheitsverlauf mit Hilfe von Genexpressionsprofilen vorherzusagen. Ein weiteres Problem bei der Krebsbehandlung ist die Vorhersage der Wirksamkeit von Krebsmedikamenten und spezifischen Ernährungsregimen, der individuell optimalen Dosierung bzw. Therapiedauer, aber auch die Vorhersage des Auftretens schwerwiegender und bedrohlicher Nebenwirkungen. Beispielsweise können genetische Polymorphismen im Gen *UGT1A1* dafür verantwortlich gemacht werden, dass bei einigen Patienten unter Therapie mit Irinotecan schwerwiegende Nebenwirkungen auftreten. Eine vorherige Genanalyse kann solche Patienten vor Therapiebeginn identifizieren und eine Dosisanpassung ermöglichen. Daneben sind Therapien mit in biotechnologischen Verfahren hergestellten Medikamenten teuer und werden zukünftig auf die Patienten beschränkt werden, bei denen eine Wirksamkeit auf Grund der individuellen Disposition wahrscheinlich erscheint. Der vorliegenden Erfindung liegt daher die Aufgabe zu Grunde, ein Mittel bereitzustellen, das eine bessere Prognose des natürlichen Verlaufs einer Krebserkrankung und das Ansprechen auf jedwede Therapieform erlaubt. Insbesondere soll dieses Mittel auch dazu in der Lage sein, diejenigen Patienten zu erkennen, bei denen spezifische Medikamente wirksam sein werden.

Krebszellen sind gekennzeichnet durch einen Verlust der Kontaktinhibition und unkontrolliertes Zellwachstum. Ausgelöst werden solche Veränderungen spontan oder durch Noxen, sog. Kanzerogene, welche das Erbgut schädigen. Zu solchen Noxen gehören viele Chemikalien, Tabakrauch, aber auch UV-Licht. Daneben spielen genetische Faktoren bei der Krebsentstehung eine herausragende Rolle. Kennzeichnend für Krebszellen ist neben ihrem ungehemmten Wachstum auch die Neigung, Tochtergeschwülste (Metastasen) in anderen Organen abzusiedeln. Die Ausbreitung der Metastasen erfolgt regelmäßig über die Blutbahn oder über Lymphgefäße. Krebserkrankungen sind bei einem Großteil der Fälle nicht heilbar und führen zum Tode. Therapeutisch wird versucht, den Ausgangstumor und Metastasen operativ zu entfernen. Daneben können Tumore bestrahlt werden. Mittels so genannter Zytostatika, Antikörper gegen bestimmte Proteine oder Oberflächenmarker oder immunmodulierender Substanzen (Zytokine, Interferone) wird versucht, die sich schnell teilenden Krebszellen abzutöten oder in den programmierten Zelltod (Apoptose) zu überführen.

Es ist von medizinisch überaus hoher Relevanz, Prognosefaktoren für den Verlauf von Krebserkrankungen zu definieren, die Auskunft über das Ansprechen auf bestimmte Therapieformen geben oder die generell prädiktiv für das Auftreten von Metastasen, die Tumorprogression und das Überleben sind. Bislang werden in der Medizin dem Fachmann allgemein bekannte Prognosefaktoren eingesetzt. Hierzu gehören beispielsweise die Größe des Tumors, seine Eindringtiefe in das umgebende Gewebe, organüberschreitendes Wachstum, das Eindringen in Blut-oder Lymphgefäße oder in Lymphknoten, sowie der Differenzierungsgrad der Tumorzellen. Daneben existieren einige relativ unspezifische serologische Marker. Das Verfahren zur Klassifikation der Tumore wird allgemein als Staging und Grading bezeichnet. Generell gilt, dass das Vorhandensein von Fernmetastasen und ein geringer Differenzierungsgrad prognostisch sehr ungünstige Parameter darstellen. Dennoch ist es die allgemeine Erfahrung in der Medizin, dass Patienten bei gleichem Tumorstadium drastisch unterschiedliche Krankheitsverläufe aufweisen. Während man bei manchen Patienten eine schnelle Progression der Erkrankung und das Auftreten von Metastasen und Rezidiven beobachtet, kommt die Erkrankung bei anderen Patienten aus unklaren Gründen zum Stillstand. Metastasen können hierbei lokal, regional und fern der Muttergeschwulst auftreten. Dazu ist es notwendig, dass eine hohe Zahl von Geschwulstzellen über den Lymph- oder Blutweg durch einfache Fortschwemmung in Flüssigkeit oder durch Abklatschen in benachbartes Gewebe gelangt. Unter Rezidivneigung versteht man das erneute Auftreten einer Geschwulst nach operativer unvollständiger oder Teilentfernung des Tumors. Hierbei handelt es sich nicht um eine erneute maligne Transformation, sondern um das Nachwachsen eines nicht vollständig entfernten Tumorgewebes. Eine Rezidivbildung ist auch durch Metastasen möglich, die viele Jahre latent bleiben können. Unter Progression versteht man das Wiederauftreten eines Tumors mit höherem Grading (mehr Entdifferenzierung) oder das Neuauftreten von Metastasen. Ganz offensichtlich gibt es eine Vielzahl individueller, unerkannter biologischer Variablen, die den Verlauf einer Tumorerkrankung unabhängig von Staging und

Grading in hohem Maße determinieren. Zu solchen Faktoren gehören genetische Wirtsfaktoren.

Daneben ist es wünschenswert, genetische Marker zu entwickeln, die prädiktiv für das Auftreten von Tumoren sind. Solche Marker erfüllen die Funktion, die betroffenen Individuen frühzeitig weiteren Screeningmaßnahmen (Serologie, Röntgen, Ultraschall, NMR etc.) zuzuführen. Damit können Krebserkrankungen im Frühstadium erkannt und therapeutisch angegangen werden, wobei die Heilungs-und Überlebenschancen bei Tumoren im Frühstadium wesentlich besser sind als bei fortgeschrittenen Tumoren.

Generell können alle Zellen des menschlichen Körpers maligne entarten und zu einer Krebserkrankung führen. Die hier und im Weiteren gemachten Ausführungen beschreiben generelle Mechanismen der Tumorprogression, der Metastasierung und des therapeutischen Ansprechens. Insofern gelten die hier beschriebenen Mechanismen und Ansprüche für alle Tumore des Menschen, beispielsweise für die folgenden Tumoren:
- Tumoren des Urogenitaltrakts: Hier sind zu nennen das Harnblasenkarzinom, das Nierenzellkarzinom, das Prostatakarzinom und das Seminom.
- Tumoren der weiblichen Geschlechtsorgane: Das Mammakarzinom, das Korpuskarzinom, das Ovarialkarzinom, das Zervixkarzinom.
- Tumoren des Gastrointestinaltraktes: Das Mundhöhlenkarzinom, das Ösophaguskarzinom, das Magenkarzinom, das Leberkarzinom, das Gallengangskarzinom, das Pankreaskarzinom, das Kolonkarzinom, das Rektumkarzinom.
- Tumore des Respirationstraktes: das Kehlkopfkarzinom, das Bronchialkarzinom.
- Tumore der Haut: das maligne Melanom, das Basaliom, das T-Zell-Lymphom.
- Tumorerkrankungen des blutbildenden Systems: Hodgkin- und Non-Hodgkin-Lymphome, akute und chronische Leukämien, Plasmozytom, etc.
- Tumorerkrankungen des Gehirns bzw. des Nervengewebes: Glioblastom, Neuroblastom, Medulloblastom, meningeales Sarkom, Astrozytom.
- Weichteiltumore, beispielsweise Sarkome und Kopf-Hals-Tumore.

Die humane Familie der Polo-like Kinasen wurde nach ihrem Ortholog *Polo* in *Drosophila melanogaster* benannt, das als erstes identifiziert worden ist. Zudem wurden weitere Orthologe in *Saccharomyces cerevisiae (Cdc5)* und *Schizosaccharomyces pombe (Plo1)* identifiziert und analysiert, wobei dieser hochkonservierten Kinasenfamilie eine entscheidende Rolle bei der mitotischen Progression spielt (van Vugt MATM et al., Oncogene 24:2844-2859 (2005)).

Im Menschen wurden bisher vier Gene, die für Polo-like Kinasen (PLK) kodieren, identifiziert: *PLK1, PLK2* (auch u.U. als *SNK [serum-inducible kinase]* bezeichnet), *PLK3* (auch u.U. als *PRK [praliferation-related kinase]* bezeichnet) und *PLK4* (auch u.U. als *SAK [Snk actin kinase]* bezeichnet). PLKs sind Serin/Threonin-Kinasen, die eine N-terminale Kinasedomäne und entweder eine oder 2 C-terminale charakteristische, repetitive Sequenzmotive, die Poloboxen, besitzen. Die Gene *PLK1, PLK2* und *PLK3* enthalten 2 Poloboxen (PB1 und PB2), währen *PLK4* nur eine Polobox besitzt, die zur Bildung von PLK4-Homodimeren beiträgt. Die Polobox-Motive sind für die subzelluläre Lokalisation im Bereich mitotischer Strukturen, für das Fortschreiten der Mitose während des Zellzyklus sowie für die Regulation der Kinaseaktivität verantwortlich. Zudem regulieren die PLKs Zytokine und die zelluläre Antwort auf DNA-Schädigungen (Eckerdt F. et al., Oncogene 24:267-276 (2005)).

Der am besten untersuchte Vertreter der Familie der Polo-like Kinasen ist PLK1. Die Expression von *PLK1* erfolgt beginnend in der S-Phase des Zellzyklus' und erreicht ihr Maximum während der G2/M-Phase. Die Kinase-Aktivität dieses Enzyms ist während der Mitose am höchsten. Beim Übergang von der G2- zur M-Phase trägt PLK1 zur Bildung der Zentrosomen, der Bildung der bipolaren Spindel, zur Adaptation des DNA-damage-Checkpoints und zur Aktivierung von MPF (maturating-promoting factor) durch die Phosphorylierung von CDC25C und Cyclin B1 bei, wobei letzteres für den Eintritt der Zelle in die Prophase der Zellteilung obligatorisch ist (Ohkura H. et al., Genes Dev. 9:1059-1073 (1995); Sanchez Y. et al., Science 286:1166-1171 (1999); Roshak A. K. et al., Cell Signal 12:405-411 (2000); do Carmo A. M. et al., Nat. Cell. Biol. 3:421-424 (2001); Yuan J. et al., Oncogene 21:8282-8292 (2002)). Diese Funktionen sind Spezies-übergreifend hoch konserviert. Eine Dysregulation der PLK1-Aktivität führt somit zu aberranten Zentrosomen und Spindelapparaten, wodurch es zu Aneuploidien und chromosomaler Instabilität kommen kann (Eckerdt F. et al., Oncogene 24:267-276 (2005)).

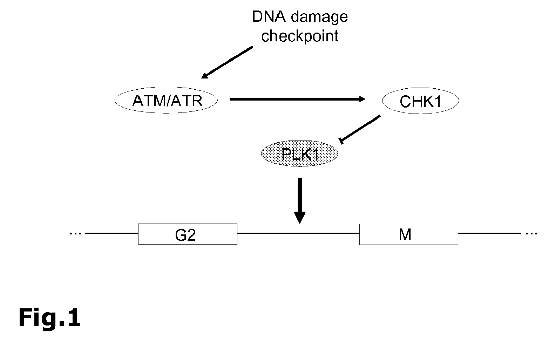

Aufgrund der Beteiligung von PLK1 an der Regulation des Zellzyklus' wurde ihre potentielle Bedeutung für die Onkogenese genauer untersucht. Die Aktivität der PLK1 wird vom *DNA damage checkpoint* bestimmt, indem es zu einer negativen Rückkopplung durch die Checkpointkinase 1 kommt. Diese Hemmung der PLK1 ist essentiell für einen effektiven G2-Arrest nach einer DNA-Schädigung (Fig. 1). Ist die PLK1-Expression hochreguliert und damit auch ihre Enzymaktivität, verkürzt sich der G2-Arrest und die Zelle tritt wieder in den Zellzyklus ein, ohne dass die Reparaturvorgänge abgeschlossen werden konnten, wodurch eine Akkumulation defekter DNA begünstigt wird (Shtivelman E., Mol. Cancer Res. 1:959-969 (2003)). Zudem ist PLK1 in der Lage, den Tumorsuppressor p53 zu inhibieren und somit auch seine proapoptotische Funktion (Ando K. et al., J. Biol. Chem. 279:25549-25561 (2004)).

Übereinstimmend mit diesen *in vitro*-Ergebnissen konnte wiederholt in verschiedenen Tumorarten eine Überexpression der *PLK1* gezeigt werden. In Schilddrüsen- und Mammakarzinomen wurden stark erhöhte PLK1-Level nachgewiesen (Salvatore G. et al., Cancer Res. 67:10148-10158 (2007); Li Y. et al., Mol. Cancer 6:7). Eine erhöhte *PLK1*-Expression wurde ebenfalls in gastrointestinalen Tumoren sowie in Prostata- und Pankreastumoren gezeigt, wobei eine gesteigerte Expression mit einem schlechteren Überleben der Patienten assoziiert war (Weichert W. et al., Prostate 60:240-245 (2004); Weichert W. et al., Pancreatology 5:259-265 (2005); Kanaji S. et al., Oncology 70:126-133 (2006)). Der Grund für die PLK1-Überexpression bei diesen Patienten ist bisher allerdings unbekannt.

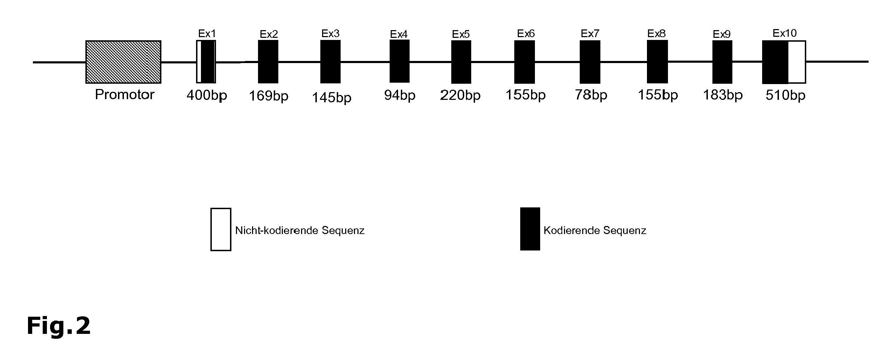

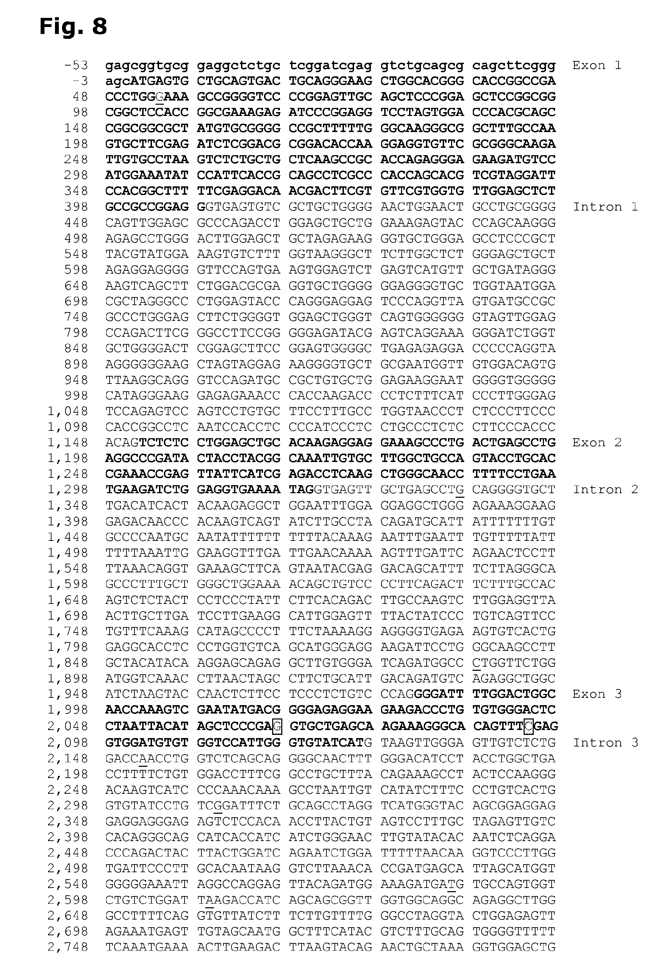

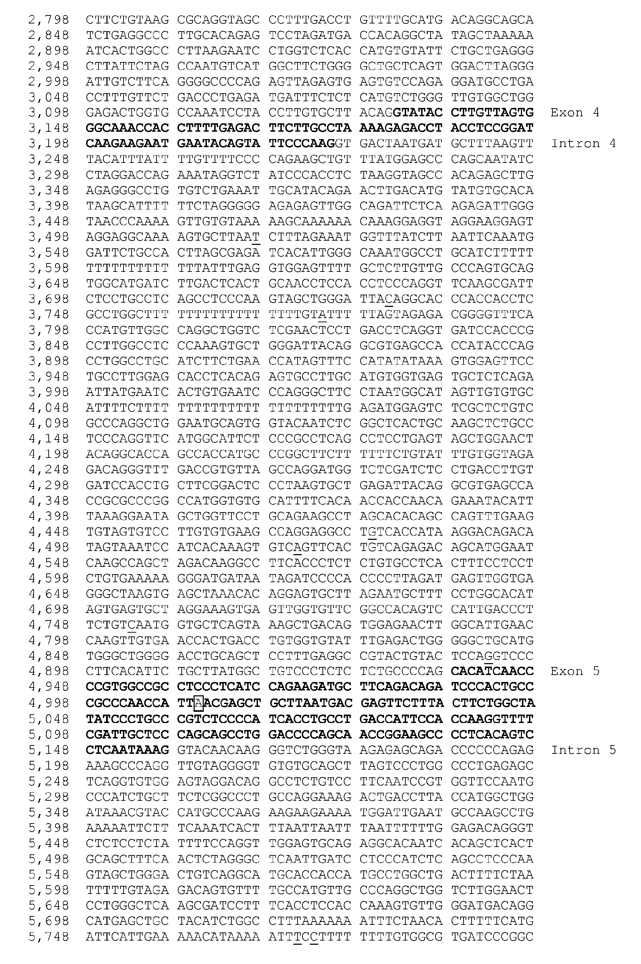

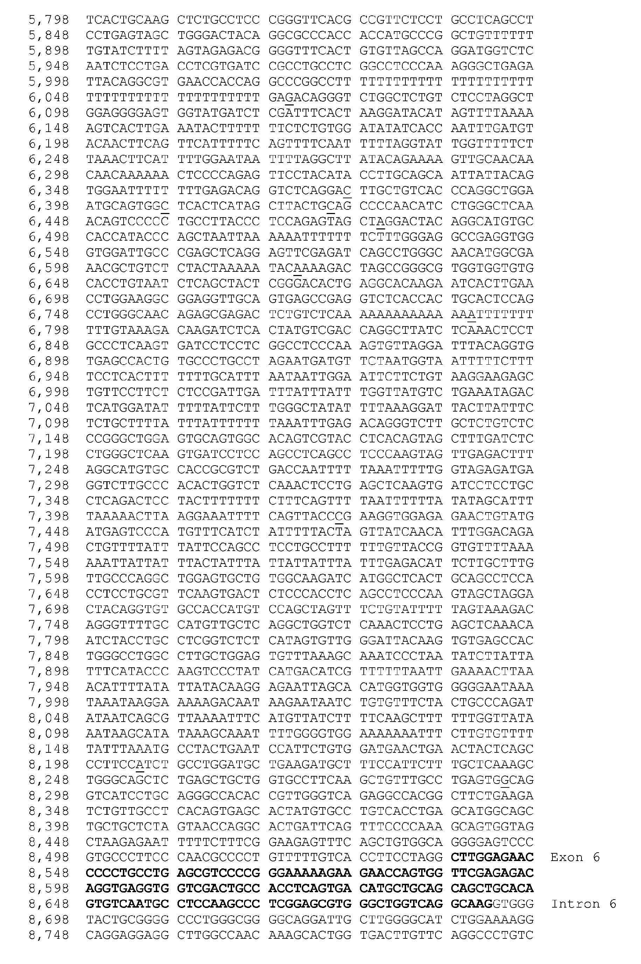

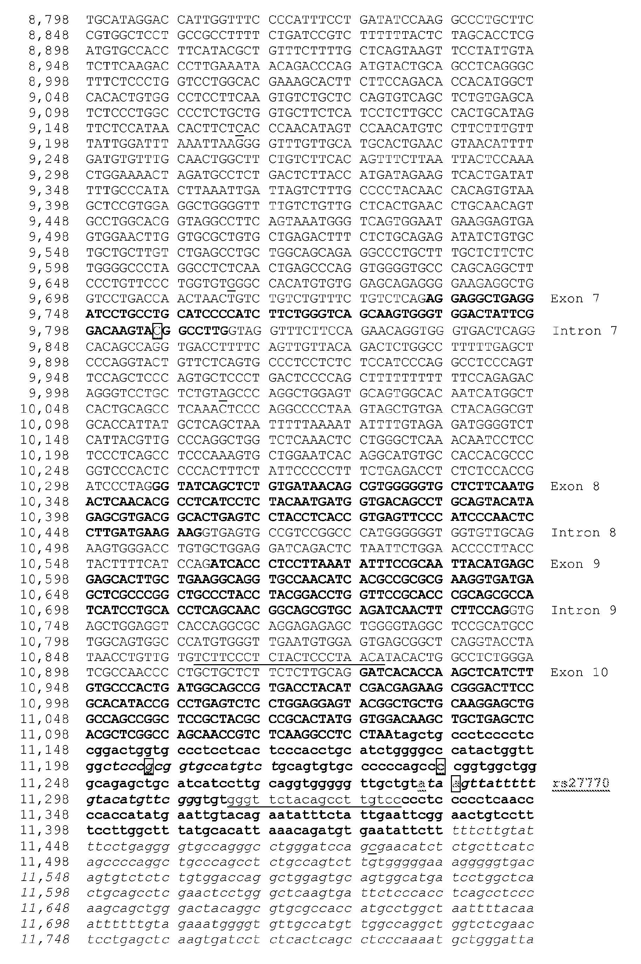

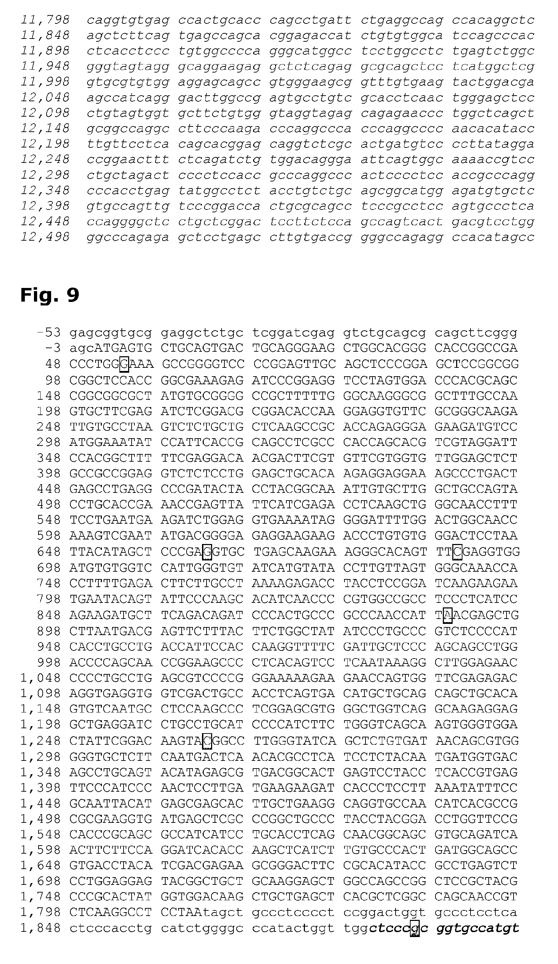

Das Gen, das für die PLK1 kodiert, ist auf Chromosom 16p12.1 lokalisiert (Gene bank accession number NM_005030; siehe Fig. 8 und SEQ ID NO:1). Es besteht aus 10 Exons und kodiert für ein 66 kDa großes Protein, das nukleär exprimiert wird. Eine schematische Darstellung der Genstruktur findet sich in Fig. 2. Der Genpromotor ist bereits identifiziert und gut charakterisiert. Er besteht aus einer core-Region und besitzt zusätzlich einen *silencer-* sowie einen enhancer-Bereich (Bräuninger A. et al., Oncogene 11:1793-1800 (1995)). Die 3'-untranslatierte Region (3'UTR) des Gens ist bisher noch nicht genauer untersucht worden.

### Kurzbeschreibung der Erfindung

Es wurde nun ein funktionsverändernder genomischer Polymorphismus in der 3'UTR des Gens *PLK1* gefunden, der mit einer Änderung der Proteinexpression verknüpft ist und der geeignet ist, generell Krankheitsrisiken und -verläufe und generell Ansprechen auf Pharmaka und Krebstherapien, insbesondere PLK1-Inhibitoren, und Nebenwirkungen vorherzusagen. Dieser Polymorphismus eignet sich weiterhin zum Auffinden und/oder Validieren weiterer Polymorphismen bzw. Haplotypen im Gen *PLK1.* Die Erfindung betrifft somit
(1) ein *in-vitro* Verfahren zur Vorhersage von Krankheitsrisiken, Krankheitsverläufen, Arzneimittelrisiken und zum Auffinden von drug targets, das das Durchmustern einer biologischen Probe nach einer oder mehreren Genveränderung(en) in der 3'UTR des Gens *PLK1,* das für das Protein Polo-like Kinase 1 kodiert, auf dem Humanchromosom 16p12.1 umfasst;
(2) ein bevorzugter Aspekt des Verfahren nach (1), wobei die biologische Probe wenigstens auf die Genveränderung(en) an Position 11.285 des in Fig. 8 gezeigten Sequenz in der 3'-untranslatierten Genregion von *PLK1*, vorzugsweise auf den Polymorphismus A11285G durchmustert wird;
(3) ein Verfahren zum Auffinden relevanter Polymorphismen und Haplotypen, im Gen *PLK1* und in dazu benachbarten Genen umfassend die Quantifizierung der Expression von PLK1 im Zusammenhang mit dem in (1) oder (2) beschriebenen Polymorphismus und Abgleich mit der Expression von anderen Polymorphismen der *PLK1;* und
(4) ein Kit zur Durchführung des Verfahrens nach (1) oder (2), umfassend Primer/Sonden, die zur Detektiondtektion des Polymorphismus Al1285G in der 3'UTR des Gens *PLK1* geeignet sind.

### Kurzbeschreibung der Figuren

Figur 1: Schematische Darstellung der Reaktionskaskade verantwortlich für den Übergang von der G2- zur M-Phase, die das Gen *PLK1* involviert.

Figur 2: Exon/Intron-Struktur des humanen Gens *PLK1* (nicht maßstabsgetreu).

Figur 3: Darstellung der beiden möglichen Allele des PLK1-3'UTR-SNPs mit flankierenden Sequenzen (SEQ ID Nos:4 und 5). Die Konsensusbindestelle für den MIBP1/RFX1-Komplex beim Vorliegen des G-Allels ist aufgezeigt.

Figur 4: Genotyp-abhängige Darstellung der PLK1-Expression relativ zu β-Actin in Nierenzellkarzinomgewebe.

Figur 5: Genotyp-abhängige Darstellung des Überlebens als Kaplan-Meier-Kurven bei Patienten mit Nierenzellkarzinom und einem Follow-up von 240 Monaten.

Figur 6: Kaplan-Meier-Analyse zum Überleben von Patienten mit Harnblasenkarzinom abhängig vom Genotyp des *PLK1*-3'UTR-Polymorphismus und einem Follow-up von 100 Monaten.

Figur 7: Kaplan-Meier-Analyse zum Überleben von Patientinnen mit Mammakarzinom abhängig vom Genotyp des *PLK1*-3'UTR-Polymorphismus und einem Follow-up von 150 Monaten.

Figur 8: Gen codierend für das Protein Polo-like Kinase 1 (SEQ IS NO:1). Die Nummerierung erfolgt ausgehend vom A des Startkodons ATG als +1. Buchstaben in Normalschrift stehen für Intronsequenzen, fette Buchstaben stehen für Exonsequenzen, fette Grossbuchstaben geben die kodierende Region wieder, fette Kleinbuchstaben geben die untranslatierten Regionen (UTRs) wieder, kursive Kleinbuchstaben stehen für die genomische Sequenz, die nach der 3'UTR folgt. Der Polymorphismus ist unterstrichen, die Sequenzen der Primer für die Sequenzierung sind unterstrichen, die Sequenzen der Primer für die Genotypisierung sind fett kursiv, wobei die eingerahmte Base durch Mutagenese ersetzt werden kann, um eine Restriktionsschnittstelle zu generieren.

Figur 9: Transkribierten mRNA-Sequenz von *PLK1* (SEQ ID NO:2). Die Nummerierung erfolgt ausgehend vom A des Startkodons ATG als +1. Grossbuchstaben geben die kodierende Region wieder. Kleinbuchstaben geben die untranslatierten Regionen (UTRs) wieder. Der Polymorphismus ist unterstrichen. Die Sequenzen der Primer für die Genotypisierung sind fett kursiv, wobei die eingerahmte Base durch Mutagenese ersetzt werden kann, um eine Restriktionsschnittstelle zu generieren.

Figur 10: Genomische Exon 10-Sequenz von *PLK1* mit flankierenden Sequenzen (SEQ ID NO:3). Die Nummerierung erfolgt ausgehend vom A des Startkodons ATG als +1. Buchstaben in Normalschrift stehen für Intronsequenzen, fette Buchstaben stehen für Exonsequenzen, fette Grossbuchstaben geben die kodierende Region und fette Kleinbuchstaben die 3'-untranslatierte Region (3'UTR) wieder. Kursive Kleinbuchstaben stehen für die genomische Sequenz, die nach der 3'UTR folgt. Der Polymorphismus ist unterstrichen.

### Detaillierte Beschreibung der Erfindung

Die vorliegende Erfindung beruht im Wesentlichen auf der Bereitstellung eines in der 3'UTR von *PLK1* befindlichen Polymorphismus, der durch systematische Sequenzierung von DNAs von Menschen gefunden wurde. Hierzu wurden Gensequenzen, die zum Exon 10 gehören, aber nicht translatiert sondern nur transkribiert werden, mittels PCR-Reaktionen amplifiziert und gemäß der Methode nach Sanger sequenziert. Die dazu erforderlichen Verfahren, z.B. das Ableiten von für die PCR-Reaktion erforderlichen Primerpaaren und die Auswahl von SequenzierPrimern sind dem Fachmann geläufig. Eine detaillierte Beschreibung des zum Auffinden des Polymorphismus verwendeten Verfahrens ist in den Beispielen beschrieben.

Diese Polymorphismus wird in dem erfindungsgemäßen *in-vitro* Verfahren der Aspekte (1) bis (3) der vorliegenden Erfindung zur Vorhersage von Krankheitsrisiken, Krankheitsverläufen, Arzneimittelrisiken und zum Auffinden von drug targets, bzw. zum Auffinden relevanter Polymorphismen und Haplotypen, im Gen *PLK1* und in dazu benachbarten Genen verwendet. Diese Verfahren umfassen dabei das Durchmustern einer biologische Probe zumindest auf die Genveränderung(en) an Position 11.285 der in Fig. 8 gezeigten Sequenz in der 3'-untranslatierten Genregion von *PLK1*, vorzugsweise auf den Polymorphismus A11285G. Dieses Durchmustern kann prinzipiell mit jedem dem Fachmann geläufigen Verfahren erfolgen. Es sind jedoch folgende Nachweisverfahren besonders bevorzugt: Durchmustern durch direkte Sequenzierung, PCR mit nachfolgender Restriktionsanalyse, reverse Hybridisierung, Dot-blot- oder slot-blot-Verfahren, Massenspektrometrie, Taqman®- oder Light-Cycler®-Technologie, Pyrosequencing®, Invader®-Technologie, Luminex-Verfahren oder Umsetzen mit DNA-Chips.

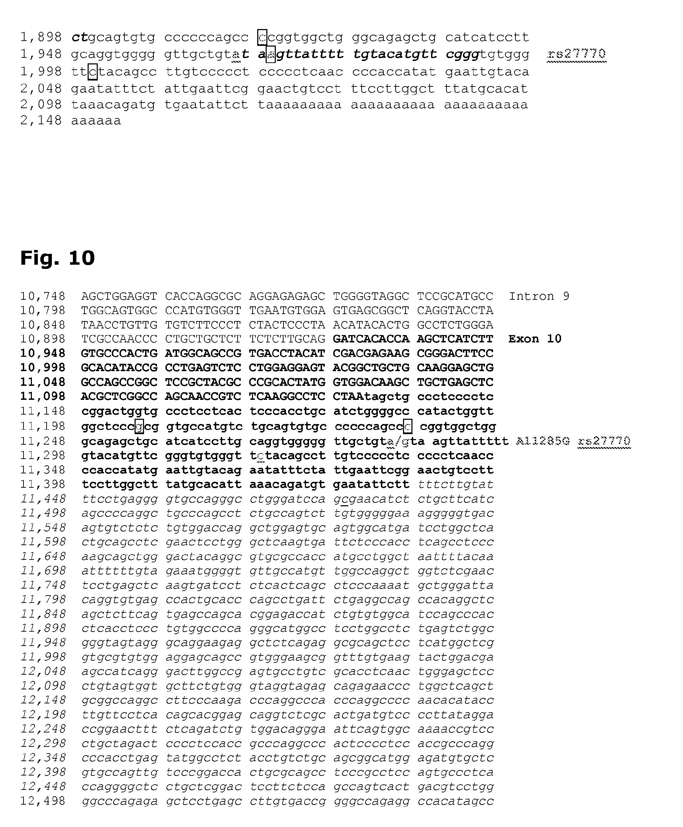

Den meisten dieser Verfahren erfordern dabei eine Amplifikationsreaktion, so z.B. mit einem Primerpaar und gegebenenfalls zusätzlichen Sequenzierprimern, die wenigstens 5, vorzugsweise wenigstens 10 zusammenhängende Nukleotide
(i) aus den Nukleotiden 10,285-11,284 und 11,286-12,285 der in Fig.9 gezeigten Sequenz, oder
(ii) aus den Nukleotiden 966-1,965 und 1,967-2,153 der in Fig.10 gezeigten Sequenz umfassen.

Der Nachweis des Polymorphismus in genomischer DNA mittels direkter Sequenzierung (z. B. nach der Sanger-Methode, der Pyrosequenzierung, der Maxam und Gilbert-Methode, der Solexa-Technologie oder Sequenzierung durch Ligation) kann mit Hilfe von Primerpaaren und/oder zusätzlichen Sequenzierprimern erfolgen, deren Sequenz aus mindestens 5 zusammenhängenden Nukleotiden, aus den Nukleotiden 10,285-11,284 und 11,286-12,285 der in Fig. 8 gezeigten Sequenz, besteht. Als Beispiel für ein solches Primerpaar sind die unterstrichenen Sequenzen in Fig. 8 genannt.

Der Nachweis des Polymorphismus mittels direkter Sequenzierung (z. B. nach der Sanger-Methode, der Pyrosequenzierung, der Maxam und Gilbert-Methode, der Solexa-Technologie oder Sequenzierung durch Ligation) in mRNA kann nach anschließender reverser Transkription in cDNA mit Hilfe von Primerpaaren und/oder zusätzlichen Sequenzierprimern erfolgen, deren Sequenz aus mindestens 5 zusammenhängenden Nukleotiden, aus den Nukleotiden 966-1,965 und 1,967-2,153 der in Fig.9 gezeigten Sequenz, besteht.

Der Nachweis des Polymorphismus in genomischer DNA mittels MALDI-TOF-MS basierter DNA-Analytik kann mit Hilfe von Primerpaaren erfolgen, deren Sequenz aus mindestens 5 zusammenhängenden Nukleotiden, aus den Nukleotiden 10,285-11,284 und 11,286-12,285 der in Fig. 8 gezeigten Sequenz, besteht. Nach anschließender Polymerasekettenreaktion (PCR) kann durch Extension eines Primers, der aus mindestens 5 zusammenhängenden Nukleotiden besteht, welche in 5'-Richtung ausgehen von Position 11,284 oder in 3'-Richtung ausgehen von Position 11,286 in der in Fig. 8 gezeigten Sequenz, der Polymorphismus über die unterschiedlichen Nukleotidmassen genotypisiert werden.

Der Nachweis des Polymorphismus mittels MALDI-TOF-MS basierter DNA-Analytik in mRNA/cDNA mit Hilfe von Primerpaaren erfolgen, deren Sequenz aus mindestens 5 zusammenhängenden Nukleotiden, aus den Nukleotiden 966-1,965 und 1,967-2,153 der in Fig. 9 gezeigten Sequenz, besteht. Nach anschließender PCR kann durch Extension eines Primers, der aus mindestens 5 zusammenhängenden Nukleotiden besteht, welche in 5'-Richtung ausgehen von Position 1,965 oder in 3'-Richtung ausgehen von Position 1,967 in der in Fig. 9 gezeigten Sequenz, der Polymorphismus über die unterschiedlichen Nukleotidmassen genotypisiert werden.

Der Nachweis des Polymorphismus in genomischer DNA mittels Restriktionsfragmentlängenpolymorphismus (RFLP) kann nach erfolgter PCR mit aus mindestens 5 zusammenhängenden Nukleotiden, aus den Nukleotiden 10,285-11,284 und 11,286-12,285 der in Fig. 8 gezeigten Sequenz, bestehenden Primern erfolgen. Der Nachweis des Polymorphismus mittels RFLP in mRNA kann nach anschließender reverser Transkription in cDNA und erfolgter PCR mit aus mindestens 5 zusammenhängenden Nukleotiden, aus den Nukleotiden 966-1,965 und 1,967-2,153 der in Fig. 9 gezeigten Sequenz, bestehenden Primern erfolgen. Zudem ist es möglich, durch einen gezielten Basenaustausch in einem Primer (Mutageneseprimer) eine Restriktionsschnittstelle zu generieren. Als Beispiel dafür dienen die fett kursiven Sequenzen Fig. 8 und 9. Hier kann durch den Austausch der Base A an Position 11,288 zu einem C die Erkennungssequenz für das Restriktionsenzym RsaI (GA/TC) erzeugt werden, d. h. *Rsa*I ist bei Vorliegen des G-Allels in der Lage zu schneiden; liegt das A-Allel vor, erfolgt keine Restriktion. Diese Vorgehensweise kann für beliebige weitere Restriktionsenzyme angewandt werden.

Der Nachweis des Polymorphismus in genomischer DNA mittels Allelspezifischer PCR kann mit Hilfe von Primerpaaren erfolgen, deren Sequenz aus mindestens 5 zusammenhängenden Nukleotiden, aus den Nukleotiden 10,285-12,285 der in Fig.8 gezeigten Sequenz, besteht. Davon muss ein Primer spezifisch für das G-Allel und das A-Allel an Position 11,285 vorliegen. Der Nachweis des Polymorphismus mittels Allelspezifischer PCR in mRNA kann nach anschließender reverser Transkription in cDNA mit Primerpaaren erfolgen, deren Sequenz aus mindestens 5 zusammenhängenden Nukleotiden, aus den Nukleotiden 966-2,153 der in Fig. 9 gezeigten Sequenz, besteht. Davon muss ein Primer spezifisch für das G-Allel und das A-Allel an Position 11,285 vorliegen.

Der Nachweis des Polymorphismus in genomischer DNA mittels Hochleistungsflüssigkeitschromatographie (HPLC) kann mit Hilfe von Primerpaaren, deren Sequenz aus mindestens 5 zusammenhängenden Nukleotiden, aus den Nukleotiden 10,285-11,284 und 11,286-12,285 der in Fig. 8 gezeigten Sequenz, besteht und anschließender PCR und dem Aufschmelzen der Amplifikate über die Bildung von Heteroduplexen erfolgen. Der Nachweis des Polymorphismus mittels HPLC in mRNA kann nach anschließender reverser Transkription in cDNA mit Primerpaaren, deren Sequenz aus mindestens 5 zusammenhängenden Nukleotiden, aus den Nukleotiden 966-1,965 und 1,967-2,153 der in Fig. 9 gezeigten Sequenz, besteht und anschließender PCR und dem Aufschmelzen der Amplifikate über die Bildung von Heteroduplexen erfolgen.

Der Nachweis des Polymorphismus in genomischer DNA mittels Allelspezifischer Hybridisierung (z. B. Taqman®- oder Light-Cycler®-Technologie) kann mit Hilfe von Primerpaaren, deren Sequenz aus mindestens 5 zusammenhängenden Nukleotiden, aus den Nukleotiden 10,285-11,284 und 11,286-12,285 der in Fig. 8 gezeigten Sequenz, besteht und anschließender PCR und dem Verwenden von Allelspezifischen Sonden erfolgen. Der Nachweis des Polymorphismus mittels Allelspezifischer Hybridisierung (z. B. Taqman®- oder Light-Cycler®-Technologie) in mRNA kann nach anschließender reverser Transkription in cDNA mit Primerpaaren, deren Sequenz aus mindestens 5 zusammenhängenden Nukleotiden, aus den Nukleotiden 966-1,965 und 1,967-2,153 der in Fig. 9 gezeigten Sequenz, besteht und anschließender PCR und dem Verwenden von Allelspezifischen Sonden erfolgen. Auch ohne vorherige PCR-Amplifikation können Allelspezifische Sonden zur Detektion des Polymorphismus eingesetzt werden (z. B. DNA- oder RNA/cDNA-Micro Arrays, DNA- oder RNA/cDNA-Blotting-Verfahren, Luminex®- oder Invader®-Technologie). Diese Sonden umfassen eine Sequenz aus mindestens 5 zusammenhängenden Nukleotiden aus den Nukleotiden 11,198-12,347 der in Fig. 8 gezeigten Sequenz oder aus den Nukleotiden 1,879-2,028 der in Fig. 9 gezeigten Sequenz, wobei sie spezifisch für das G- bzw. das A-Allel des Polymorphismus an genomischer Position 11,285 bzw. an RNA/cDNA-Position 1,966 sind.

Es ist dabei besonders bevorzugt in den vorstehenden Verfahren, dass die Primer wenigstens 10 zusammenhängende Nukleotide, vorzugsweise 10 bis 30 Nukleotide der Sequenzen von Fig. 8 bzw. Fig. 9 aufweisen. Besonders bevorzugt sind Primer, die die Sequenz von SEQ ID NO:6 bis 9, Nukleotide 10860 - 10880 und 11314 - 11332 der in Fig. 8 gezeigten Sequenz oder Nukleotide 1881 - 1899 und 1967 - 1991 der in Fig. 9 gezeigten Sequenz aufweisen.

Die vorstehend eingesetzten Sonden weisen wenigstens 10, vorzugsweise wenigstens 20 zusammenhängende Nukleotide der Sequenzen von Fig. 8 bzw. Fig. 9 auf.

Die Primer und Sonden können falls erforderlich, Markermoleküle tragen (Fluoreszenzmarker, Enzyme, Antigene, Radioisotope usw.), die einen Nachweis des Amplifikationsproduktes bzw. des Hybridisierungskomplexes ermöglichen.

Mittels der vorstehend definierten Verfahren wird ein Polymorphismus detektiert, wobei in der 3'UTR an Position 11285 ein Basenaustausch von Adenin zu Guanin vorliegt (rs27770). In Fig. 10 ist dieser Polymorphismus mit seinen flankierenden Sequenzen dargestellt. Die Nummerierung der Position erfolgt in der Weise, dass dem Nukleotid A des Startkodons ATG die Nummer +1 zugeordnet wird.

Zur Bestimmung der Verteilung des PLK1-3UTR-Polymorphismus in der Normalbevölkerung und Verwendung der Genotypen zum Auffinden weiterer relevanter Polymorphismen und Haplotypen wurden zunächst unterschiedliche DNA-Proben von gesunden Kaukasiern (n=96) genotypisiert. Das Ergebnis ist in der folgenden Tabelle 1 dargestellt:

**Tabelle 1: Verteilung des PLK1-Polymorphismus in Kaukasiern deutschen Ursprungs**

| Genotyp | Kaukasier |
|---|---|
| AA | 49 (51.0%) |
| AG | 41 (42.1%) |
| GG | 6 (6.3%) |

Ein Gegenstand der Erfindung ist es, dass der detektierte Polymorphismus dazu benutzt werden kann, um weitere relevante genomische Genveränderungen in *PLK1* oder benachbarten Genen zu identifizieren und zu validieren, die z.B. mit Genotypen im Gen *PLK1* im Kopplungsungleichgewicht stehen. Dies können auch Gene sein, die ebenfalls auf Chromosom 16 liegen, aber in großer Entfernung vom Gen *PLK1.* Hierzu wird folgendermaßen vorgegangen:
1. Für bestimmte Phänotypen (zelluläre Eigenschaften, Krankheitszustände, Krankheitsverläufe, Arzneimittelansprechen usw.) wird zunächst eine Assoziation mit den Genotypen des *PLK1*-Polymorphismus hergestellt.
2. Für neu detektierte Genveränderungen in *PLK1* oder benachbarten Genen wird untersucht, ob bereits bestehende Assoziationen unter Verwendung oben beschriebener Genotypen verstärkt oder abgeschwächt werden.

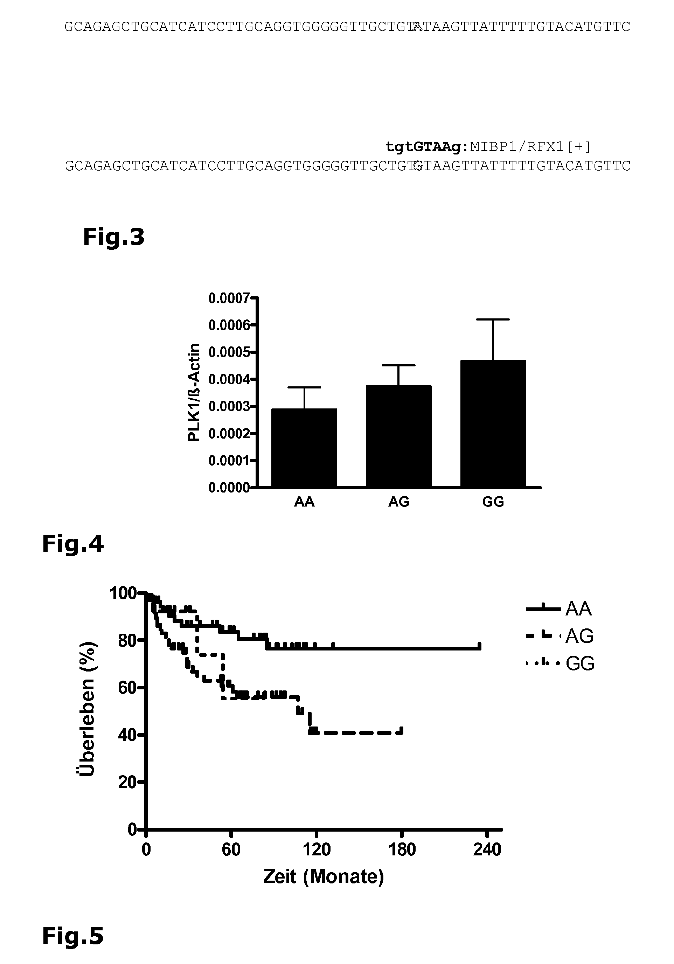

Zur Bestimmung der funktionelle Bedeutung des 3'UTR-Polymorphismus im Gen *PLK1* wurde untersucht, welche funktionellen Änderungen dem 3'UTR-Polymorphismen im Gen *PLK1* zuzuordnen sind. Hierzu wurde zunächst mittels eines Computer-Programms untersucht, ob die Basensubstitution die Bindung von Transkriptionsfaktoren oder anderen regulatorischen Proteinen verändern kann. Diese Proteine binden an spezifische Konsensus-Sequenzen in der 3'UTR von Genen und können dadurch die Stabilität der mRNA beeinflussen. Wie in Fig. 3 dargestellt, befindet sich der 3'UTR-SNP in der Konsensus-Sequenz der Bindungsstelle für den Proteinkomplex MIBP1/RFX1. Nur wenn das G-Allel an Position 11.285 vorliegt, kommt es zu einer spezifischen Bindung, nicht aber beim A-Allel.

Des Weiteren wurde untersucht, ob die Expression von *PLK1* auf mRNA-Ebene Genotyp-abhängig unterschiedlich ist. Hierfür wurde mRNA aus menschlichem Operationsgewebe bei Nierenzelloperationen gewonnen und mittels reverser Transkriptase in cDNA umgeschrieben. Das Verfahren ist dem Fachmann geläufig. Nachfolgend wurde das Expressionsniveau mittels Realtime-PCR (Taqman-Verfahren) bestimmt und mit dem Expressionsniveau des Housekeeping-Gens β-*Actin* abgeglichen. Die Ergebnisse sind in den Fig. 4 dargestellt. Der GG-Genotyp des A11285G-SNPs weist eine höhere mRNA-Expression auf als der AA-Genotyp. Die Werte des heterozygoten Genotyps liegen dazwischen, was für einen Gen-Dosis-Effekt spricht.

Damit wurde nachgewiesen, dass es im Gen *PLK1* Genveränderungen gibt, die eine Expressionsänderung von PLK1 im Karzinom-Gewebe bewirken. Dies kann der oben beschriebene 3'UTR-Polymorphismen sein oder Polymorphismen, die mit diesem SNP im Kopplungsungleichgewicht stehen. Bestandteil der hier beschrieben Erfindung ist damit auch, die Expression von PLK1 zu quantifizieren, mit bekannten Polymorphismen der *PLK1* zu assoziieren und neue, noch besser geeignete Polymorphismen zu entdecken und zu validieren.

Die hier gezeigten Befunde einer Genotyp-abhängigen Expression von PLK1 in humanem Karzinomgewebe ist bedeutend, da eine sehr hohe Aktivität von PLK1 aberrante Zentrosomen und Spindelapparate hervorrufen kann, wodurch Aneuploidien und chromosomale Instabilität begünstigt werden und sich negativ auf die Tumorprogression auswirken. Zudem kann sich diese Genotyp-abhängige Genexpression von *PLK1* auch auf das Ansprechen auf eine Therapie mit PLK1-Inhibitoren auswirken. Es ist zu erwarten, dass eine niedrige Genexpression prädisponiert durch einen bestimmten Genotyp, z.B. der AA-Genotyp des A11285G-Polymorphismus, stärker auf PLK1-Inhibitoren anspricht als die anderen Genotypen. Damit können Genveränderungen im Gen *PLK1* dazu verwendet werden, das Ansprechen auf eine Krebstherapie vorherzusagen, um zum Beispiel Responder versus Non-Responder zu diskriminieren. Diese Genveränderungen können auch für eine Dosisfindung eingesetzt werden bzw. für die Vorhersage des Auftretens unerwünschter Arzneimittelwirkungen. Solche Krebstherapien können im weitesten Sinne medikamentös erfolgen, d.h. durch Zuführung von Substanzen in den Körper oder diese Krebstherapeutika können physikalisch wirksam sein.

Wir erwarten somit die Beeinflussung von Krankheitsverläufen, insbesondere bei Tumorerkrankungen, sowie ein geändertes Ansprechen auf Substanzen, die die Regulationskaskade von PLK1 beeinflussen oder Substanzen, die direkt PLK1 inhibieren.

Aufgrund der Schlüsselfunktion von PLK1 für die Mitose und den gesamten Zellzyklus, der in Tumoren dysreguliert vorliegen kann, ist es ein wesentlicher Bestandteil der Erfindung, dass unter Verwendung von Genveränderungen in *PLK1* generell Erkrankungsrisiken und Krankheitsverläufe vorhergesagt werden können.

Die Multistep-Entwicklung von Krebs spiegelt die Akkumulation genetischer Veränderungen wider, die zur Transformation von normalen Zellen zu Krebszellen und von normalem Gewebe zu benignen und evtl. malignen, invasiven Tumoren führen. Die Ansammlung von Alterationen beschleunigt die Tumorgenese und kann auch die Therapie beeinflussen. Es wird allerdings auch deutlich, dass gestörte Mechanismen der Zellzyklusregulation der Grund für die erhöhte genomische Instabilität von Tumoren sind (Hoeijmaker J. H., Nature 411:366-74 (2001); Khanna et al., Nat. Gent. 27:247-54 (2001)). Da promitotische Kinasen eine zentrale Rolle für die Erhaltung der genomischen Integrität besitzen, ist unmittelbar zu erwarten, dass der Verlauf vielfältiger und ganz unterschiedlicher Tumorerkrankungen bei einer genetisch determinierten, verminderten Aktivierbarkeit dieser Kinasen beeinflusst wird (Nigg E. A., Curr. Biology 10:776-783 (1998); Eckerdt F. et al., Oncogene 24:267-276 (2005); van Vugt MATM et al., Oncogene 24:2844-2859 (2005); Li J. J. et al., Pharmacol. Ther. 111:974-984 (2006)). Dies bedeutet, dass durch Expressionsänderungen von Proteinen, die in allen menschlichen Körperzellen exprimiert werden und die Zellteilung regeln, Zellfunktionen bestimmt werden, die alle physiologischen und pathophysiologischen Vorgänge entscheidend beeinflussen oder zumindest modulieren. Daneben werden auch Antworten auf Pharmaka in besonderer Weise beeinflusst. Hiervon sind erwünschte, aber auch unerwünschte Arzneimittelwirkungen betroffen.

Als Erkrankungen, die mit einer Genveränderung in *PLK1* einhergehen, können genannt werden:
1. benigne Neoplasien jedes Ursprungsgewebes
2. maligne Neoplasien jedes Ursprungsgewebes

Der Kit von Aspekt (4) der Erfindung enthält vorzugsweise die vorstehend definierten Primer und Sonden. Daneben kann der Kit weitere funtionelle Komponenten wie z.B. Restriktionsenzyme, Polymerasen, Label, Vergleichssubstanzen, Puffer usw. enthalten.

Ein wesentlicher Bestandteil der vorliegenden Erfindung ist die Bereitstellung diagnostisch relevanter Genveränderungen im Gen *PLK1* als Prognosefaktor für Tumorerkrankungen des Menschen und Kits für deren Nachweis. Naturgemäß können hierbei nicht alle Tumorerkrankungen beschrieben werden. Das Prinzip wird daher an ausgewählten Beispielen erläutert, welche die generelle Verwendbarkeit demonstrieren, jedoch den Schutzbereich der Anmeldung nicht einschränken.

### Beispiele

### Beispiel 1: Detektion und Genotypisierung des Polymorphismus

Der Polymorphismus 11,285A>G (rs27770) wurde durch die direkte Sequenzierung des Exons 10 von *PLK1* detektiert. Dazu wurden folgende Primer verwendet:
5'-TCTTCCCTCTACTCCCTAACA- 3' (SEQ I D NO:6)
5'-GGACAAGGCTGTAGAACCC-3' (SEQ ID NO:7).

Die PCR-Reaktion wurde in einem Ansatz, der einen kommerziellen PCR-Mastermix (2x Taq DNA Pol Master Mix, Ampliqon), je 10 pM Primer und 50 ng genomische DNA enthielt, durchgeführt. Die Konditionen für die PCR-Reaktion waren folgende:

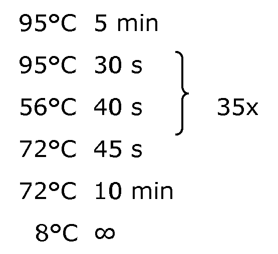

Das 473 bp große PCR-Produkt wurde mittels einer Agarose-Gelelektrophorese (1,5%) überprüft und anschließend mit Hilfe des QIAquick® PCR Purification Kit (Qiagen) nach Herstellerangaben aufgereinigt. Die Sequenzierung wurde von der Firma Eurofins Medigenomix GmbH (Martinsried) durchgeführt.

Der Polymorphismus 11,285A>G (rs27770) wurde mittels Restriktionsfragmentlängenpolymorphismus (RFLP) in Kontrollen und Patienten genotypisiert. Einer der dafür verwendeten Primer stellt einen Mutagenese-Primer dar, bei dem eine Base ausgetauscht wurde (blau hervorgehoben), um eine Restiktionsschnittstelle für das Enzym *Rsa*I einzuführen, wenn das G-Allel des Polymorphismus vorliegt. Dazu wurden folgende Primer verwendet:
5'-CTCCCGCGGTGCCATGTCT-3' (SEQ ID NO:8)
5'-CCGAACATGTACAAAAATAACGTA-3' (SEQ ID NO:9).

Die PCR-Reaktion wurde in einem Ansatz, der einen kommerziellen PCR-Mastermix (2x Taq DNA Pol Master Mix, Ampliqon), je 10 pM Primer und 50 ng genomische DNA enthielt, durchgeführt. Die Konditionen für die PCR-Reaktion waren folgende:

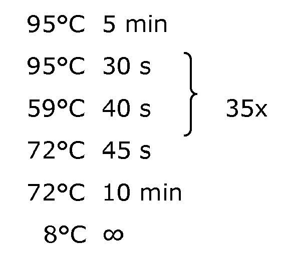

Das 110 bp große PCR-Produkt wurde mittels einer Agarose-Gelelektrophorese (1,5%) überprüft und anschließend in die RFLP-Reaktion eingesetzt, die 1 U des Enzyms *Rsa*I und den dazugehörigen Reaktionspuffer (10x; New England BioLabs) enthielt. Die Restriktion erfolgte bei 37°C für 12 Stunden. Danach wurde der Reaktionsansatz mittels Agarose-Gelelektrophorese (2,5%) aufgetrennt und ausgewertet. Es ergab sich folgendes Restriktionsmuster:

| | | | |
|---|---|---|---|
| AA | 110 bp → 10 bp + | | 100 bp |
| AG | 110 bp → 10 bp + 13 bp + 87 bp + 100 bp | | |
| GG | 110 bp → | 13 bp + 87 bp | |

### Beispiel 2: Nierenzellkarzinom

Nierenkrebs ist eine schwerwiegende Krankheit. Die Heilungschancen sind abhängig von der Tumorgröße und von der Tumorausbreitung. Bei Patienten ohne Metastasen beträgt die 10-Jahres-Überlebensrate bis zu 80%, allerdings mit einer erheblichen interindividuellen Variabilität. Durch den heute weit verbreiteten Einsatz von Ultraschalltechnik werden viele der Tumoren bereits in einem frühen nicht metastasierten Stadium erkannt und können so rechtzeitig behandelt werden. Bei einem Vorliegen von Fernmetastasen ist es möglich, die operative Entfernung der Niere mit einer anschließenden Immuntherapie mit Interleukin-2 oder Interferon-Alpha zu kombinieren. Dadurch wird die körpereigene Krebszellabwehr gestärkt. Beim metastasierten Nierenzellkarzinom kann mit der Kombination von Interferon, Interleukin-2 und 5-Fluorouracil in Studien eine Ansprechrate von 36% erreicht werden. Derzeit gibt es keine prädiktiven Marker für das Überleben bei Patienten mit Nierenzellkarzinom.

Fig. 5 zeigt das Überleben in Abhängigkeit vom *PLK1-Genotyp.* Patienten mit dem AA-Genotyp überleben deutlich länger als Patienten mit den AG- und GG-Genotypen. Die mediane Zeit bis zum Tod beträgt bei den AG- und GG-Trägern 115 Monate. Für Patienten mit dem AA-Genotyp kann das mediane Überleben nicht angegeben werden, da über den gesamten Beobachtungszeitraum von 240 Monaten weniger als die Hälfte dieser Patienten sterben.

### Beispiel 3: Harnblasenkarzinom

Blasenkrebs ist ein bösartiger Tumor der Schleimhaut der Harnblase und tritt am häufigsten zwischen dem 60. und 70. Lebensjahr auf. Männer sind davon dreimal so häufig betroffen wie Frauen. Bei Männern ist Blasenkrebs nach Lungen- und Prostatakrebs die dritthäufigste Krebsform. Blasenkrebs kann durch äußere Einflüsse hervorgerufen werden. Zu den Risikofaktoren gehören, Rauchen, ständige Belastung des Organismus durch Chemikalien, wie beispielsweise Farbstoffe oder Schmerzmittelmissbrauch. Bei vielen Patienten ergeben die Untersuchungen, dass es sich um einen oberflächlichen Tumor handelt. Dieser kann operativ mit Hilfe des Zystoskops entfernt werden. Mehr als 70% der Patienten, die wegen eines oberflächlichen Blasenkarzinoms behandelt wurden, zeigen im Verlauf ein Tumorrezidiv. Dabei kommen in mehr als der Hälfte Rezidivtumoren mit nichtmuskelinvasiver Erkrankung vor. Diese können durch transurethrale Resektion kurativ behandelt bzw. kontrolliert werden. Es ist deshalb wichtig, diese Läsionen frühzeitig zu erkennen und den Patienten regelmäßig und engmaschig nachzusorgen. In regelmäßigen Intervallen dienen Ausscheidungsurogramme zur Kontrolle möglicher Tumormanifestationen in Nierenbecken und Harnleitern. Bislang gibt es kaum valide Marker, die für den weiteren Verlauf der Erkrankung prädiktiv sind. Derzeit werden daher die klassischen Faktoren wie Eindringtiefe, Differenzierungsgrad, Metastasierung, Lymphknotenbefall etc. für die Prognose herangezogen. Genetische Marker für Überlebenswahrscheinlichkeit und Therapieansprechen würden die Betreuung von Patienten mit Harnblasenkarzinom wesentlich verbessern. Aufgabe der Erfindung ist es zu zeigen, dass die Verwendung von Genveränderungen in *PLK1* dazu geeignet ist, den weiteren Verlauf der Erkrankung vorherzusagen.

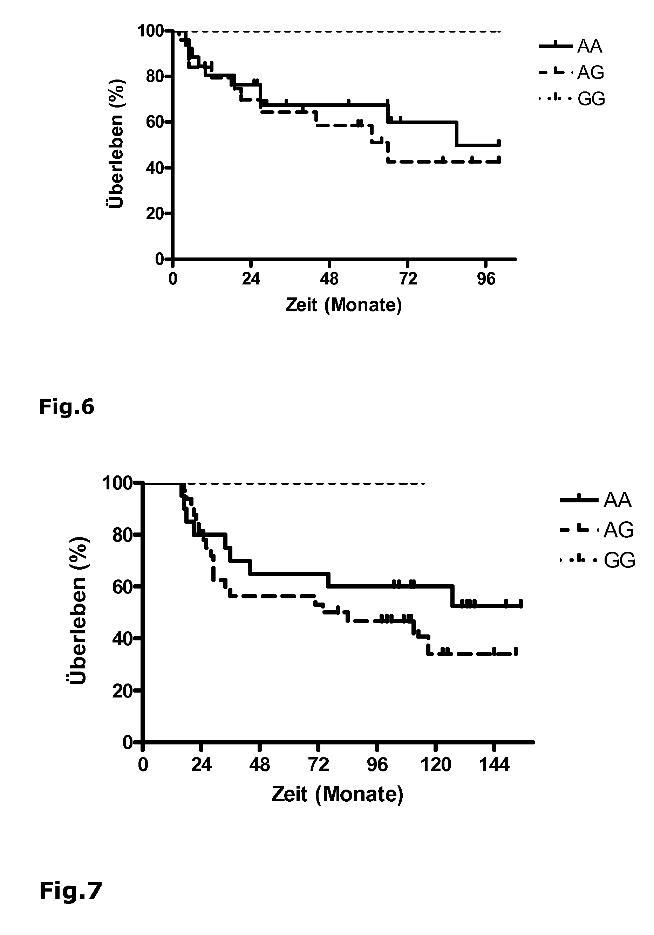

Fig. 6 zeigt das Überleben von Patienten mit Harnblasenkarzinom in Abhängigkeit vom *PLK1-*Genotyp. Hierbei sind deutliche Unterschiede für das Überleben der Patienten zu sehen. Patienten mit dem GG-Genotyp zeigen das beste Überleben. Die mediane Zeit bis zum Tod beträgt 87 Monate beim GG-Genotyp und 66 Monate beim AG-Genotyp. Beim AA-Genotyp kann keine mediane Zeit angegeben werden, da bis zum Ende der Studie weniger als die Hälfte dieser Patienten verstarben.

### Beispiel 4: Mammakarzinom

Das Mammakarzinom ist der häufigste Tumor der weiblichen Bevölkerung in Europa und den USA. Es betrifft 7 -10% aller Frauen und macht 25% der gesamten weiblichen Krebsmortalität aus. Die Ätiologie des Mammakarzinoms ist noch nicht bekannt, jedoch sind Risikofaktoren beschrieben, wie eine familiäre Disposition, Strahlenexposition oder Östrogeneinfluss. Bei den meisten Patientinnen ergeben die Untersuchungen, dass es sich um ein invasives Karzinom handelt. Mit wenigen Ausnahmen wird jedes operable Mammakarzinom selbst bei nachgewiesener Fernmetastasierung chirurgisch behandelt. Die unterschiedlich radikale operative Erstversorgung hat Variationen der lokoregionären Rezidivrate, nicht aber der langfristigen Überlebenschance zur Folge. Zudem können Rezidive oder Fernmetastasen nicht selten erst nach 5 oder sogar 10 Jahren manifest werden. Es ist deshalb wichtig, diese Läsionen frühzeitig zu erkennen und die Patientinnen engmaschig nachzusorgen. Nachsorgeuntersuchungen werden in regelmäßigen Intervallen durchgeführt, bei zwischenzeitlichem Verdacht auch bis zu 10 Jahre postoperativ. Bislang gibt es kaum valide Marker, die für den weiteren Verlauf der Erkrankung prädiktiv sind. Derzeit werden daher die klassischen Faktoren wie Tumorgröße, Metastasierung, Lymphknotenbefall, Hormonrezeptorstatus etc. für die Prognose herangezogen. Genetische Marker für Überlebenswahrscheinlichkeit und Therapieansprechen würden die Betreuung von Patientinnen mit Mammakarzinom wesentlich verbessern. Aufgabe der Erfindung ist es zu zeigen, dass die Verwendung von Genveränderungen in *PLK1* dazu geeignet ist, den weiteren Verlauf der Erkrankung vorherzusagen.

Fig. 7 zeigt das Überleben von Mammakarzinom-Patientinnen in Abhängigkeit vom Genotyp. Patientinnen mit dem GG-Genotyp zeigen das beste Überleben verglichen mit Patientinnen, die mindestens ein A-Allel tragen. Die mediane Zeit bis zum Tod für den AG-Genotyp beträgt 79 Monate. Bei den anderen beiden Genotypen kann kein medianes Überleben angegeben werden, da hier weniger als die Hälfte der Patientinnen während des gesamten Beobachtungszeitraumes sterben.

### Beispiel 5: Verwendung von Genveränderungen im Gen PLK1 zur Vorhersage von Krankheitsverläufen und Therapieansprechen

Da die essentiellen Funktionen von PLK1 bekannt sind, können Genveränderungen im Gen *PLK1* das Risiko für Tumorerkrankungen erhöhen bzw. Krankheitsverläufe beeinflussen. Es ist generell nicht möglich, alle Tumorerkrankungen des Menschen und deren Verläufe zu untersuchen. Wir haben dies hier jedoch exemplarisch für drei unterschiedliche Karzinome gezeigt: Nierenzellkarzinom, Harnblasenkarzinom und Mammkarzinom. Diese Daten belegen eindeutig die Verwendbarkeit der Genveränderungen im Gen *PLK1* für den hier beschriebenen Zweck. Diese Erkrankungen stehen *a priori* in keinerlei Zusammenhang.

### Beispiel 6: Pharmakogenetik - Diagnostik der Wirksamkeit von Pharmaka, der Potenz und Effizienz von Pharmaka und dem Auftreten unerwünschter Wirkungen

Grundlagen und Ziele der Pharmakogenetik: Die Wirksamkeit von Arzneimitteln und/oder das Auftreten unerwünschter Nebenwirkungen wird neben den spezifischen Stoffeigenschaften der chemisch definierten Produkte durch eine Reihe von Parametern definiert. Zwei wichtige Parameter, die erzielbare Plasmakonzentration und die Plasmahalbwertszeit, bestimmen in hohem Maß die Wirksamkeit oder Unwirksamkeit von Pharmaka oder das Auftreten unerwünschter Wirkungen. Die Plasmahalbwertszeit wird unter anderem dadurch bestimmt, mit welcher Geschwindigkeit bestimmte Pharmaka in der Leber oder anderen Körperorganen zu wirksamen oder unwirksamen Metaboliten verstoffwechselt und mit welcher Geschwindigkeit sie aus dem Körper ausgeschieden werden, wobei die Ausscheidung über die Nieren, über die Atemluft, über den Schweiß, über die Spermaflüssigkeit, über den Stuhl oder über andere Körpersekrete erfolgen kann. Daneben wird die Wirksamkeit bei oraler Gabe durch den sog. "first-pass-Effekt" limitiert, da nach Resorption von Pharmaka über den Darm ein bestimmter Anteil in der Leber zu unwirksamen Metaboliten verstoffwechselt wird.

Mutationen oder Polymorphismen in Genen metabolisierender Enzyme können die Aktivität derselben in der Weise verändern, dass deren Aminosäurezusammensetzung verändert wird, wodurch die Affinität zum zu metabolisierenden Substrat erhöht oder erniedrigt wird und damit der Metabolismus beschleunigt oder verlangsamt sein kann. In ähnlicher Weise können Mutationen oder Polymorphismen in Transportproteinen die Aminosäurezusammensetzung in der Weise verändern, dass der Transport und damit die Ausscheidung aus dem Körper beschleunigt oder verlangsamt wird.

Zur Auswahl der für einen Patienten optimal geeigneten Substanz, der optimalen Dosierung, der optimalen Darreichungsform und zur Vermeidung unerwünschter, z. T. gesundheitsschädlicher oder tödlicher Nebenwirkungen ist die Kenntnis von genetischen Polymorphismen oder von Mutation, die zur Änderung der Genprodukte führen, von herausragender Bedeutung.

Polokinase-Inhibitoren: Aufgrund der promitotischen Funktion von PLK1 und der erhöhten PLK1-Expression in verschiedenen Tumoren, wird die Inhibierung der PLK1 als viel versprechende Therapiestrategie angesehen. Die Herunterregulation von *PLK1* durch siRNA in Nacktmäusen mit Xenografttumoren führte zu einem geringeren Tumorwachstum (Spänkuch B. et al., J. Natl. Cancer Inst. 96:862-872 (2004)). Zudem konnte gezeigt werden, dass die Inhibierung der PLK1 Tumorzellen der Apoptose zuführt, während normale Zellen in ihrer Funktion kaum verändert werden, was für ein günstiges Nebenwirkungsprofil potentieller PLK1-Inhibitoren spricht (Liu X. et al., Mol. Cell. Biol. 26:2093-2108 (2006)). Auch die ersten niedermolekularen PLK1-Inhibitoren, wie das Benzylstyrylsulfon-Analogon ON01910 und dem Dihydropteridinon-Derivat BI2536 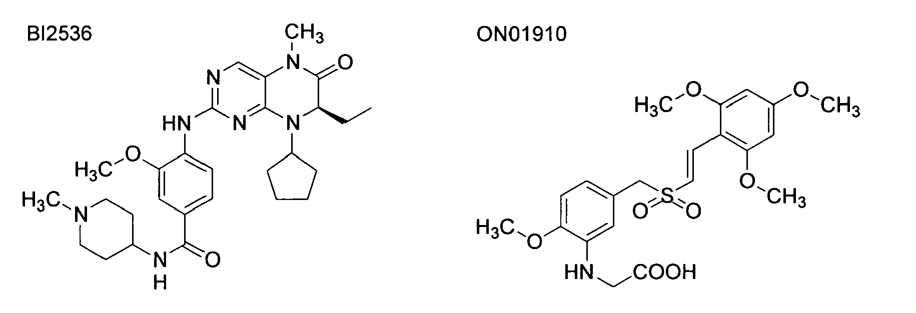 (siehe Strebhardt, K. et al., Nat. Rev. Cancer 6:321-330 (2006)), führten zu einem verlangsamten Tumorwachstum. Sie könnten sich als gute Ergänzung oder Substitution der bisherigen Mitosehemmern (z.B. Colchicinalkaloide und Vinca) erweisen (Gumireddy K. et al., Cancer Cell. 7:275-286 (2005); Steegmaier M. et al., Curr. Biology 17:316-322 (2007)).

### Seqenzprotokoll, freier Text

| | |
|---|---|
| SEQ ID NO:1 | Gen codierend für das Protein Polo-like Kinase 1. |
| SEQ ID NO:2 | Transkribierten mRNA-Sequenz von *PLK1* |
| SEQ ID NO:3 | Genomische Exon 10-Sequenz von *PLK1* mit flankierenden Sequenzen |
| SEQ ID NO:4 und 5 | Darstellung der beiden möglichen Allele des *PLK1-*3'UTR-SNPs mit flankierenden Sequenzen.. |
| SEQ ID NOs:6 bis 9 | Primer |

## Patentansprüche

1. *In-vitro* Verfahren zur Vorhersage von Krankheitsrisiken, Krankheitsverläufen, Arzneimittelrisiken und zum Auffinden von drug targets, das das Durchmustern einer biologischen Probe nach einer oder mehreren Genveränderung(en) in der 3'UTR des Gens *PLK1,* das für das Protein Polo-like Kinase 1 kodiert, auf dem Humanchromosom 16p12.1 umfasst.

2. Verfahren nach Anspruch 1, wobei die biologische Probe wenigstens auf die Genveränderung(en) an Position 11.285 des in SEQ ID NO:1 gezeigten Sequenz in der 3'-untranslatierten Genregion von *PLK1,* vorzugsweise auf den Polymorphismus A11285G durchmustert wird.

3. Verfahren nach Anspruch 1 oder 2, wobei das Durchmustern durch direkte Sequenzierung, PCR mit nachfolgender Restriktionsanalyse, reverse Hybridisierung, Dot-blot- oder slot-blot-Verfahren, Massenspektrometrie, Taqman®- oder Light-Cycler®-Technologie, Pyrosequencing®, Invader®-Technologie, Luminex-Verfahren oder Umsetzen mit DNA-Chips erfolgt.

4. Verfahren nach Anspruch 3, das eine Amplifikationsreaktion mit einem Primerpaar und gegebenenfalls zusätzlichen Sequenzierprimern, die wenigstens 5 zusammenhängende Nukleotide
(i) aus den Nukleotiden 10,285-11,284 und 11,286-12,285 der in Fig.9 gezeigten Sequenz, oder
(ii) aus den Nukleotiden 966-1,965 und 1,967-2,153 der in Fig.10 gezeigten Sequenz umfasst.

5. Verfahren nach Anspruch 3 oder 4, das aus einen oder mehrere der folgenden Verfahren ausgewählt ist
(i) direkte Sequenzierung in genomischer DNA, umfassend Umsetzung mit wenigstens einem Primerpaar und gegebenenfalls zusätzlichen Sequenzierprimern, die wenigstens 5 zusammenhängende Nukleotide aus den Nukleotiden 10,285-11,284 und 11,286-12,285 der in Fig. 8 gezeigten Sequenz umfassen;
(ii) direkte Sequenzierung nach reverser Transkription in cDNA, umfassend Umsetzung mit wenigstens einem Primerpaar und gegebenenfalls zusätzlichen Sequenzierprimern, die wenigstens 5 zusammenhängende Nukleotiden aus den Nukleotiden 966-1,965 und 1,967-2,153 der in Fig. 9 gezeigten Sequenz umfassen;
(iii) Nachweis in genomischer DNA mittels MALDI-TOF-MS, umfassend PCR mit wenigstens einem Primerpaar, das wenigstens 5 zusammenhängende Nukleotide aus den Nukleotiden 10,285-11,284 und 11,286-12,285 der in Fig. 8 gezeigten Sequenz umfasst, Extension mit einem Primer, der wenigstens 5 zusammenhängende Nukleotide aufweist, welche in 5'-Richtung ausgehen von Position 11,284 oder in 3'-Richtung ausgehen von Position 11,286 der in Fig. 8 gezeigten Sequenz, und Genotypisierung des Polymorphismus über die unterschiedlichen Nukleotidmassen;
(iv) Nachweis des Polymorphismus mittels MALDI-TOF-MS in mRNA/cDNA, umfassend PCR mit wenigstens einem Primerpaar, das wenigstens 5 zusammenhängende Nukleotide aus den Nukleotiden 966-1,965 und 1,967-2,153 der in Fig. 9 gezeigten Sequenz umfasst, Extension mit einem Primer, der wenigstens 5 zusammenhängende Nukleotide aufweist, welche in 5'-Richtung ausgehen von Position 1,965 oder in 3'-Richtung ausgehen von Position 1,967 in der in Fig. 9 gezeigten Sequenz, und Genotypisierung des Polymorphismus über die unterschiedlichen Nukleotidmassen ;
(v) Nachweis des Polymorphismus in genomischer DNA mittels Restriktionsfragmentlängenpolymorphismus (RFLP), umfassend PCR mit wenigstens einem Primerpaar, das wenigstens 5 zusammenhängende Nukleotide aus den Nukleotiden 10,285-11,284 und 11,286-12,285 der in Fig. 8 gezeigten Sequenz umfasst, reverser Transkription in cDNA, PCR mit wenigstens einem Primerpaar, das wenigstens 5 zusammenhängende Nukleotide aus den Nukleotiden 966-1,965 und 1,967-2,153 der in Fig. 9 gezeigten Sequenz umfasst, optional in Gegenwart eines Mutageneseprimers, und RFLP Bestimmung;
(vi) Nachweis des Polymorphismus in genomischer DNA mittels allelspezifischer PCR, umfassend Umsetzung mit wenigstens einem Primerpaar, das wenigstens 5 zusammenhängende Nukleotide aus den Nukleotiden 10,285-12,285 der in Fig. 8 gezeigten Sequenz aufweist, wobei ein Primer spezifisch für das G-Allel und einer spezifisch für das A-Allel an Position 11,285 ist;
(vii) Nachweis des Polymorphismus mittels allelspezifischer PCR in mRNA, umfassend reverse Transkription in cDNA, Umsetzung mit wenigstens einem Primerpaar, das wenigstens 5 zusammenhängende Nukleotide aus den Nukleotiden 966-2,153 der in Fig. 9 gezeigten Sequenz aufweist, wobei ein Primer spezifisch für das G-Allel und ein Primer spezifisch für das A-Allel an Position 11,285 ist;
(viii) Nachweis des Polymorphismus in genomischer DNA mittels HPLC, umfassend PCR mit wenigstens einem Primerpaar, das wenigstens 5 zusammenhängende Nukleotide aus den Nukleotiden 10,285-11,284 und 11,286-12,285 der in Fig. 8 gezeigten Sequenz aufweist, Aufschmelzen der Amplifikate und HPLC Nachweis über die Bildung von Heteroduplexen;
(ix) Nachweis des Polymorphismus mittels HPLC in mRNA, umfassend reverse Transkription in cDNA, PCR mit wenigstens einem Primerpaar, das wenigstens 5 zusammenhängende Nukleotide aus den Nukleotiden 966-1,965 und 1,967-2,153 der in Fig. 9 gezeigten Sequenz aufweist, Aufschmelzen der Amplifikate und HPLC Nachweis über die Bildung von Heteroduplexen;
(x) Nachweis des Polymorphismus in genomischer DNA mittels Allelspezifischer Hybridisierung umfassend Umsetzung mit allelspezifischen Sonden und optional vorrausgehende PCR Amplifikation der genomischen DNA mit wenigstens einem Primerpaar, das wenigstens 5 zusammenhängende Nukleotide aus den Nukleotiden 10,285-11,284 und 11,286-12,285 der in Fig. 8 gezeigten Sequenz aufweist; und
(xi) Nachweis des Polymorphismus mittels Allelspezifischer Hybridisierung in mRNA umfassend reverser Transkription in cDNA, und Umsetzung mit allelspezifischen Sonden, und optional vorrausgehende PCR Amplifikation der cDNA mit wenigstens einem Primerpaar, das wenigstens 5 zusammenhängende Nukleotide aus den Nukleotiden 966-1,965 und 1,967-2,153 der in Fig. 9 gezeigten Sequenz aufweist;
wobei die allelspezifischen Sonden in (x) und (xi) Sequenzen wenigstens 10 zusammenhängende Nukleotide aus den Nukleotiden 11,198-12,347 der in Fig. 8 gezeigten Sequenz oder aus den Nukleotiden 1,879-2,028 der in Fig. 9 gezeigten Sequenz aufweisen und wobei die Sonden spezifisch für das G- oder das A-Allel des Polymorphismus an genomischer Position 11,285 bzw. an RNA/cDNA-Position 1,966 sind.

6. Verfahren nach Anspruch 5, wobei
(i) die Primer wenigstens 10 zusammenhängende Nukleotide, vorzugsweise 10 bis 30 Nukleotide der Sequenzen von Fig. 8 bzw. Fig. 9 aufweisen und vorzugsweise die Sequenz von SEQ ID NO:6 bis 9, Nukleotide 10860 - 10880 und 11314 -11332 der in Fig. 8 gezeigten Sequenz oder Nukleotide 1881 - 1899 und 1967 - 1991 der in Fig. 9 gezeigten Sequenz aufweisen; und/oder
(ii) die Sonden wenigstens 20 zusammenhängende Nukleotide der Sequenzen von Fig. 8 bzw. Fig. 9 aufweisen.

7. Verfahren nach einem oder mehreren der Ansprüche 1 bis 6,
(i) das zur Voraussage von Krebsrisiken oder dem Verlauf einer Krebserkrankung, einschließlich Nierenzell-, Harnblasen- und Mammakarzinom ... geeignet ist, wobei ein AA Genotyp die beste Überlebensprognose zeigt; oder
(ii) das zum Auffinden und Evaluieren von Krebstherapeutika geeignet ist; oder
(iii) in dem Hemmstoffe der Polo-like Kinase 1, insbesondere das Benzylstyryl sulfonderivat ON01910 oder das Dihydropteridinonderivat BI2536 eingesetzt werden.

8. Verfahren zum Auffinden relevanter Polymorphismen und Haplotypen, im Gen *PLK1* und in dazu benachbarten Genen umfassend die Quantifizierung der Expression von PLK1 im Zusammenhang mit dem in Anspruch 1 oder 2 beschriebenen Polymorphismus und Abgleich mit der Expression von anderen Polymorphismen der *PLK1.*

9. Kit zur Durchführung des Verfahrens nach Anspruch 1 bis 7 umfassend Primer und/oder Sonden, die zur Detektiondtektion des Polymorphismus A11285G in der 3'UTR des Gens *PLK1* geignet sind.

10. Kit nach Anspruch 9, wobei
(i) die Primer und Sonden wie in Anspruch 4 bis 6 beschrieben sind, und/oder
(ii) der Kit weiterhin Restriktionsenzyme, Polymerasen, Label, Vergleichssubstanzen, Puffer usw. enthält.
